# NEW EUROPEAN PATENT SPECIFICATION

(11) **EP 0 201 540 B2**
(45) Date of publication and mention of the opposition decision: **31.10.2001**
(45) Mention of the grant of the patent: 19.06.1991
(21) Application number: 85905513.9
(22) Date of filing: 18.10.1985
(51) Int. Cl.: C07K 14/16

(54) **ENVELOPE ANTIGENS OF LYMPHADENOPATHY ASSOCIATED VIRUS AND THEIR APPLICATIONS**
LYMPHADENOPATHIE-ASSOZIIERTE HÜLLENANTIGENE UND DEREN VERWENDUNGEN
ANTIGENES D'ENVELOPPE DU VIRUS DES LYMPHADENOPATHIES ET LEURS APPLICATIONS

(30) Priority: 18.10.1984 FR 8416013; 16.11.1984 GB 8429099; 21.01.1985 GB 8501473
(43) Date of publication of application: 20.11.1986
(60) Divisional application: 90105189.6
(73) Proprietor: INSTITUT PASTEUR, 75724 Paris Cédex 15 (FR); CENTRE NATIONAL DE LA RECHERCHE SCIENTIFIQUE (CNRS), 75700 Paris Cedex 07 (FR)
(72) Inventor: MONTAGNIER, Luc, F-92350 Le Plessis Robinson (FR); KRUST, Bernard, F-75012 Paris (FR); CHAMARET, Solange, F-75015 Paris (FR); CLAVEL, François, F-75016 Paris (FR); CHERMANN, Jean-Claude, F-78310 Elancourt (FR); BARRE-SINOUSSI, Françoise, F-92130 Issy-Les-Moulineaux (FR); ALIZON, Marc, F-75005 Paris (FR); SONIGO, Pierre, F-75015 Paris (FR); STEWART, Cole, F-92320 Chatillon (FR); DANOS, Olivier, F-75015 Paris (FR); WAIN-HOBSON, Simon, F-78180 Montigny Les Bretonneux (FR)
(74) Representative: Gutmann, Ernest
(86) International application number: EP8500548
(87) International publication number: WO8602383

(56) References cited:
- EP-A- 0 113 078
- EP-A- 0 115 459
- EP-A- 0 178 978
- WO-A-84/04327
- WO-A-87/02988
- US-A- 4 520 113
- BIOTECHNOLOGY, vol. 4, March 1986, p. 166
- THE LANCET, 9.6.84, p. 8389
- SCIENCE, vol. 233, 11.7.86, pp. 209-212
- PROTEIN ENGINEERING, vol. 2, no. 3, 1988, pp. 219-225
- AIDS, 1988, vol. 2, no. 1, pp. 17-24
- AIDS, 1988, vol. 2, no. 3, pp. 165-169
- PROC.NATL.ACAD.SCI. USA, vol. 78. no. 6, June 1981, pp. 3403-3407
- Science, vol. 224, 4 May 1984, (US); J. Schüpbach et al.:"Serological analysis of a subgroup of human T-lymphotropic retroviruses (HTLV) associated with AIDS, pp. 503-505
- SCIENCE, vol. 225, 20 July 1984, (US); V.S. Kalyanaraman et al.:"Antibodies to the core protein of lymphadenopathy-associated virus (LAV) in patients with AIDS", pp. 321-323
- SCIENCE, vol. 228, 3 May 1985, (US); W.G. Robey et al.:"Characterization of envelope and core structural gene products of HTLV-III with sera from AIDS, patients", pp. 593-595
- SCIENCE, vol. 228, 31 May 1985, US; J.S. Allan et al.:"Major Glycoprotein antigens that induce antibodies in AIDS patients are encoded by HTLV-III", pp. 1091-1094
- Chemical Abstracts, vol. 103, no. 19, 11 November 1985, Columbus, Ohio, US; R. Crowl et al.:"HTLV-III env gene products synthesized in E. coli are recognized by antibodies present in the sera of AIDS patients" , p. 190, abstract no. 1554983e
- Current Biotechnology Abstracts, 0310003226, Centocor unveils rDNS AIDS assay; faces FDA, 5 August 1985, US; Biotechnology Newswatch, vol. 5, issue 15, p. 3
- Chemical Abstracts, vol. 103, no.5, 5 August 1985, Columbus, Ohio, US; L. Montagnier et al.:"Identification and antigenicity of the major envelope glycoprotein of lymphadenopathy-associated virus", p. 263, abstract no. 34641v
- NATURE, vol. 316, 4 July 1985; R.A. Weiss et al.:"Neutralization of human T-lymphotropic virus type III by sera of AIDS and AIDS-risk patients", pp. 69-72
- SCIENCE, vol. 228, 31 May 1985, US; F. Barin et al.:"Virus envelope protein of HTLV-III represents major target antigen for antibodies in AIDS patients", pp. 1094-1096
- CELL, vol. 40, January 1985, Cambnridge, Mass., US; S. Wain-Hobson et al.:"Nucleotide sequence of the AIDS virus, LAV", pp. 9-17
- NATURE, vol. 313, 7 February 1985, MA. Muesing et al.:"Nucleic acid structure and expression of the human AIDS/lymphadenopathy retrovirus", pp. 450-458
- Chemical Abstracts, vol. 103, no. 7, 19 August 1985, Columbus, Ohio, US; J. Schneider et al.:""A glycopolypeptide (gp 100) is the main antigen detected by HTLV-III antiserums", p. 430, abstract no. 52370k
- Chemical Abstracts, vol. 103, no. 15, 14 October 1985, Columbus, Ohio, US; M.G. Sarngadharan et al.:"Immunological properties of HTLV-IIIantigens recognized by sera of patients with AIDS and AIDS-related complex and of asymptomatic carriers of HTLV-III infection", p. 551, abstract no. 121329t
- NATURE, vol. 312, 20/27 December 1984, London, GB; M. Alizon et al.:"Molecular cloning of lymphadenopathy-associated virus", pp. 757-760
- BIOTECHNOLOGY, vol. 3, October 1985; T.W. Chang et al.:"Detection of antibodies to human T-cell lymphotropic virus-III (HTLV-III) with an immunoassay employing a recombinant escherichia coli-derived viral antigenic peptide", pp. 905-909
- NATURE, vol. 315, 9 May 1985, London, GB; N.T. Chang et al.:"An HTLV-III peptide produced by recombinant DNA is immunoreactive with sera from patients with AIDS", pp. 151-154
- NATURE, vol.295, no. 5845, 14 January 1982, GB; G.R. Dreesman et al.:"Antibody to hepatitis B surface antigen after a single inoculation of uncoupled synthetic HBsAg peptides", pp. 158-160

## Description

The present invention relates to antigens, particularly in a purified form, of the virus of lymphadenopathies (denoted below by the abbreviation LAS) and of the acquired immuno-depressive syndrome (denoted below by the abbreviation AIDS), to a process for producing these antigens, particularly antigens of the envelopes of these viruses. The invention also relates to polypeptides, whether glycosylated or not, encoded by said DNA sequences.

The causative agent if LAS or AIDS, a retrovirus, has been identified by F. BARRE-SINOUSSI et al, Science, 220, 868 (1983). It has the following characteristics. It is T-lymphotropic; its prefered target is constituted by Leu 3 cells (or T4 lymphocytes) ; it has reverse transcriptase activity necessitating the presence of Mg + and exhibits strong affinity for poly(adenylate-oligodeoxy-thymidylate)(poly(A)-oligo(dT)-12-18).
It has a density of 1.16-1.17 in a sucrose gradient, an average diameter of 139 nanometers; and a nucleus having an average diameter of 41 nanometers. Antigens of said virus, particularly a protein p25 are recognised immunologically by antibodies contained in serums taken up from patients afflicted with LAS or AIDS. The p25 protein, which is a core protein, is not recognised immunologically by the p24 protein of the HTLVI and II viruses. The virus is also free of a p19 protein which is immunologically cross-reactive with the p19 proteins of HTLVI and HTLVII.

Retroviruses of this type (sometimes denoted by the generic abbreviation LAV) have been filed in the National Collection of Micro-organism Cultures of the INSTITUT PASTEUR of Paris, under numbers I-232, I-240 and 1-241. Virus strains similar to LAV in all respects from the morphological and immunological point of view have been isolated in other laboratories. Reference is made by way of examples to the retrovirus strains named HTLV-III isolated by R.C. GALLO et al., Science, 224, 500 (1984) and by M.G. SARNGAD-HARAN et al., Science 224, 506 (1984) respectively and to the retrovirus isolated by M. JAY LEVY et al., Science, 225, 840-842 (1984), which virus was designated ARV. For the ease of language the last mentioned viruses, as well as others which have equivalent morphological and immunological properties, will be designated hereafter under the generic designation "LAV". Reference is also made to European patent application filed 14 September 1984, with the priority of British patent application number 83 24800 filed 15 september 1983 as regards a more detailed description of the LAV retroviruses or the like and of the uses to which extracts of these viruses give rise.

Initially the core antigens were the main antigens of the virus lysates or extracts which were recognised by serums of patients infected with AIDS or LAS, in the test systems which had then been used. A p42 protein, presented as consisting of an envelope protein, had been detected too. In the same manner GALLO et al disclosed a p41 protein which was also deemed to be on a possible component of the viru envelope.

Processes for obtaining a LAV virus have also been described. Reference may be made particularly to the article already mentioned of F. BARRE-SINOUSSI et al., as regards the preparation of the virus in T lymphocyte cultures derived either from blood, or from the umbilical cord, or also from bone marrow cells of adult donors in good health. This process comprises particularly the following essential steps :
- producing a viral infection of these T lymphocytes, after activation by a lectin mitogen, with a viral suspension derived from a crude supernatant liquor of lymphocytes producing the virus (initially obtained from a patient infected with AIDS or LAS),
- culturing cells infected with TCGF, in the presence of anti-α-interferon sheep serum,
- effecting purification of the virus produced (production starts generally between the 9th and the 15th day following infection and lasts from 10 to 15 days), which purification comprises precipitating the virus in polyethylenglycol in order to produce a first concentration of the virus, then centrifugating the preparation obtained in a 20-60 % sucrose gradient or in an isotonic gradient of metrizanide (sold under the trade mark NYCODENZ by NYEGAARD, Oslo) and recovering the virus with the band having a density of 1.16-1.17 in the sucrose gradient or of 1.10-1.11 in the NYCODENZ® gradient.

The LAV virus may also be produced from permanent cell lines of type T, such as the CEM line, or from B lymphoblastoid cell lines, such as obtained by the transformation of the lymphocytes derived from a healthy donor with the Epstein-Barr virus, for instance as disclosed in EP-A-0165120. The permanent cell lines obtained produce continuously a virus (designated as LAV-B in the case of the B lymphoblastoid cell lines) which possesses the essential antigenic and morphological features of the LAV viruses (except that it is collected in a density band sometimes slightly higher than in the preceding case (particularly 1.18) in sucrose. The final purification of the virus can also be carried out in a NYCODENZ® gradient.

Reference is made to the article of Schüpbach et al, Science, vol. 224, pages 503-505, 4 May 1984. This article discloses a work with respect to different antigens of the HTLV-III retrovirus.

Results are presented including a reference to an antigen having migrated on an electrophoresis gel at a migration distance corresponding to a molecular weight of approximately 110,000.

Schüpbach et al mentioned that this antigen was detected in a virus preparation but was below limit of detection in the cells.

A method for cloning DNA sequences hybridizable with the genomic RNA of LAS has already been disclosed in EP-A-0178978. Reference is hereafter made to that application as concerns subject matter in common with the further improvements to the invention disclosed herein.

The present invention further aims at providing additional new means which should not only also be useful for the detection of LAV or related viruses (hereafter more generally referred to as "LAV viruses"), but also have more versatility, particularly in detecting specific parts of the genomic DNA of said viruses whose expression products are not always directly detectable by immunological methods. The present invention further aims at providing polypeptides containing sequences in common with polypeptides comprising antigenic determinants included in the proteins encoded and expressed by the LAV genome occuring in nature. An additional object of the invention is to further provide means for the detection of proteins related to LAV virus, particularly for the diagnosis of AIDS or pre-AIDS or, to the contrary, for the detection of antibodies against the LAV virus or proteins related therewith, particularly in patients afflicted with AIDS or pre-AIDS or more generally in asymtomatic carriers and in blood-related products. Finally the invention also aims at providing immunogenic polypeptides, and more particularly protective polypeptides for use in the preparation of vaccine compositions against AIDS or related syndroms.

The present invention relates to additional DNA fragments, hybridizable with the genomic RNA of LAV as they will be disclosed hereafter, as well as with additional cDNA variants corresponding to the whole genomes of LAV viruses. It further relates to DNA recombinants containing said DNAs or cDNA fragments.

An unaltered purified LAV retrovirus distinguishes from those which have been defined above, in that it includes an amount of one or several envelope antigens, sufficient to be visualized when the virus is labelled with ³⁵S-cystein, free of unlabelled cystein in a proportion of 200 microcuries per ml of medium. these antigens, among which particularly glycoproteins, are recognised selectively in vitro by serums of patients affected with SIDA or SLAs or by the serums of asymptomatic carriers of the virus.

A preferred antigen according to the preceding definition obtainable from a lysate of this virus (or by gentle scouring of the envelopes of the virus) is a glycoprotein having a molecular weight of the order of 110,000 daltons, as determined by its migration distance in comparison with the distances of migrations, in a same migration system, of standard proteins having known molecular weights. Particularly comparative measurements were made on a 12.5 % polyacrylamide gel under a voltage of 18 V for 18 hours, upon using the following standard proteins (marketed by AMERSHAM) :
- lysozyme-(¹⁴C)-methyl (MW: 14,300),
- carbon dioxide-(¹⁴C)-methyl (MW: 30,000),
- ovalbumin-(¹⁴C)-methyl (MW: 46,000),
- bovin albumin serum (¹⁴C)-methyl (MW: 69,000),
- phosphorylase b-(¹⁴C)-methyl (MW: 92,500),
- myosine-(¹⁴C)-methyl (MW: 200,000).

The invention relates also to the antigens themselves, particularly that of molecular weight of about 110,000-120,000, which possess also the capability of being recognised by serums of patients infected with AIDS or LAS or by serums of persons who have been exposed to LAV viruses or those analogous with the latter. These antigens have also the characteristic of forming complexes with concanavaline A, said complex being dissociatable in the presence of O-methyl-α-D-mannopyranoside. The antigens according to the invention can also bind to other lectins for example those known under the name "LENTYL-LECTIN". The preferred antigen according to the invention, of molecular weight 110,000, is also sensitive to the action of endoglycosidases. This action is manifested by the production from the antigen of molecular weight 110,000 of a protein having a molecular weight of the order of 90,000, the latter being separable for example by immunoprecipitation or by separation employing the differences in molecular weights (migrations differentiated on gel).

Preferred antigens of the invention are constituted by glycoproteins.

The invention relates also to the process for producing the viruses according to the invention. This process distinguishes essentially from those recalled above at the level of the final purification operation. In particular, the purification step of the process according to the invention is no longer carried out in gradients, but involves the performance of differential centrifugations effected directly on the supernatants of the culture media of the producing cells. These centrifugation operations comprise particularly a first centrifugation at an angular centrifugation velocity, particularly of 10,000 rpm, enabling the removal of non-viral constituents, more particularly of cellular constituents, then a second centrifugation at higher angular velocity, particularly at 45,000 rpm, to obtain the precipitation of the virus itself. In preferred embodiments, the first centrifugation at 10,000 rpm, is maintained for 10 minutes and the second at 45,000 rpm, for 20 minutes. These are, of course, only indicative values, it being understood that it remains within the ability of the specialist to modify the centrifugation conditions, to provide for the separation of the cellular constituents and of the viral constituents.

This modification of the purification process results in the production of viral preparaations from which the antigen mentioned can then be isolated more easely, than from virus preparations purified by the previous methods. In any event, the viruses finally obtained by the process of the present invention are more easely recognised by serums of patients or of persons who have been exposed to the LAV virus or to morphologically and antigenically similar strains.

The antigens according to the invention can themselves be obtained from the above disclosed viruses, by lysis (or other suitable processing) of the latter in the presence of any suitable detergent and by recovery and separation of the antigens released. Advantageously, the lysis of the virus is effected in the presence of aprotinin or of any other agent suitable for inhibiting the action of proteases. The separation of the antigens according to the invention can then be carried out by any method known in itself ; for example, it is possible to proceed with a separation of the proteins by employing their respectively different migrations in a predetermined gel, the protein sought being then isolated from the zone of the gel in which it would normally be found in an electrophoresis operation under well determined conditions, having regard to its molecular weight. The antigens according to the invention can however be separated from the lysate of the abovesaid viruses, due to their affinity for lectins, in particular concanavaline A or lentyl-lectin. The lectin used is preferably immobilised on a solid support, such as the cross linked polymer derived from agarose and marketed under the trade mark SEPHAROSE. After washing of the fixed antigens with a suitable buffer, the antigens can be eluted in any suitable manner, particularly by resorting to a O-methyl-α-D-mannopyranoside in solution.

A more thorough purification of these antigens can be performed by immunoprecipitation with the serums of patients known to possess antibodies effective against said protein, with concentrated antibody preparations (polyclonal antibodies) or again with monoclonal antibodies, more particularly directed against the antigen according to the invention, in particular that having the molecular weight of 110,000, denoted below by the abbreviation gp110.

Additional characteristics of the invention will appear also in the course of the description which follows of the isolation of a virus according to the invention and of antigens, particularly an envelope antigen of the virus. reference will be made to the drawings in which :
Figure 1 is derived from a photographic reproduction of gel strips which have been used to carry out electrophoreses of lysate extracts of T lymphocytes, respectively infected and uninfected (controls) by a LAV suspension.
Figures 2 and 3 are restriction maps of a complete LAV genome (clone λJ19) or of fragments thereof.
Figures 4 to 4 are the complete sequence of a LAV viral genome.
Figures 5 and 6 show diagrammatically parts of the three possible reading phases of LAV genomic RNA, including the open reading frames (ORF) apparent in each of said reading phases.

### I - PRODUCTION OF THE VIRUS AND OF ANTIGENS

T lymphocytes derived from a healthy donor and infected with LAV1, under the conditions described by F. BARRE-SINOUSSI et Coll., on CEM cells derived from a patient afflicted with leukemia and also infected in vitro with LAV1, were kept under cultivation in a medium containing 200 microcuries of ³⁵S-cystein and devoid of unlabelled cystein. The infected lymphocytes were cultured in a non denaturating medium to prevent the degradation of the antigen sought. The supernatant liquor from the culture medium was then subjected to a first centrifugation at 10,000 rpm for 10 minutes to remove the non viral components, then to a second centrifugation at 45,000 rpm for 20 minutes for sedimenting the virus. the virus pellet was then lysed by detergent in the presence of aprotinin (5 %) particularly under the conditions described in the article of F. BARRE-SINOUSSI et Coll.

The same operation was repeated on lymphocytes taken up from a healthy donor as control.

The various lysates were then immuno-precipitated by serums of patients infected with AIDS or with LAS. Serums originating from healthy donors or of donors infected with other diseases were immunoprecipitated too. The media were then subjected to electrophoreses in a SDS-polyacrylamide gel.

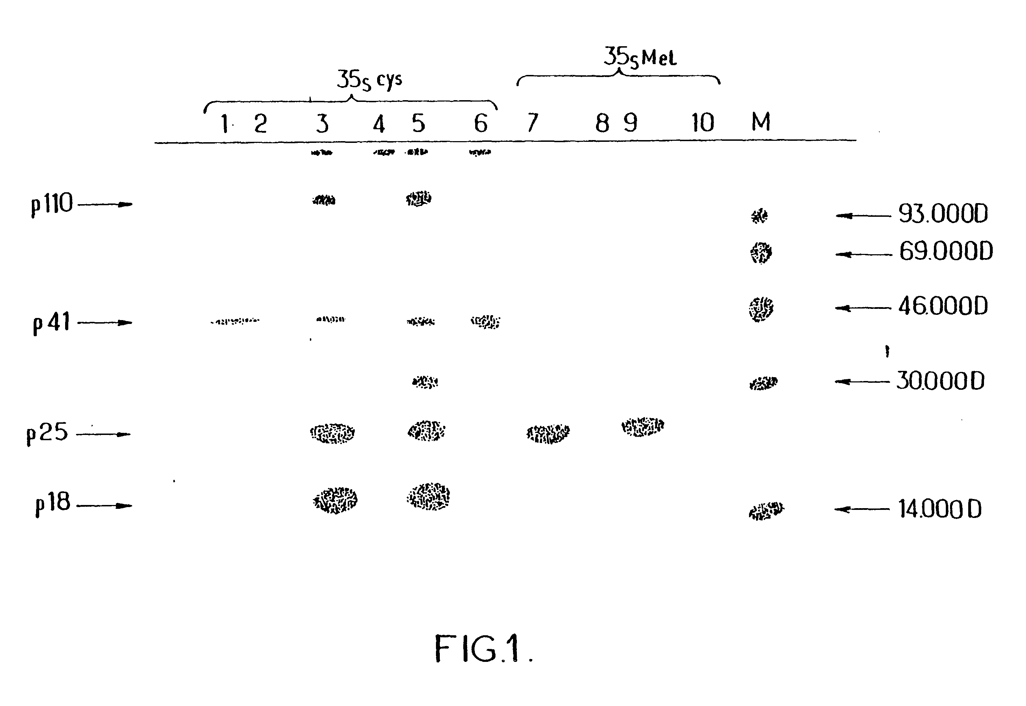

The results are indicated in figure 1. The gel strips numbered from 1 to 6 were obtained from preparations labelled by ³⁵S-cystein. The strips numbered 7 to 10 show results observed on infected or uninfected lymphocyte preparations labelled with ³⁵S-methionine. Finally the strip M corresponds to the migration distances of the standard proteins identified above, whose molecular weights are recalled in the right hand portion of the figure.

The references to the labelled viral proteins appear on the left handside of the figure.

It is noted that columns 7 to 10 show the specific protein p25 of LAV, labelled with ³⁵S-methionin. The same protein is absent on strips 8 to 10 corresponding to results obtained with a preparation originating from healthy lymphocytes.

Columns 3 and 5 correspond to the results which have been observed on preparations obtained from lymphocytes infected and labelled with ³⁵S-cystein. The proteins p25 and p18, the characteristic core proteins of LAV, and the glycoprotein gp110, also specific of LAV, were also present. Images corresponding to a protein p41 (molecular weight of the order of 41,000) appeared in the various preparations, although less distinctly.

The virus according to the invention and the antigen according to the invention can be either precipitated by lectins, particularly concanavaline A, or fixed to a SEPHAROSE®-concanavaline A column. Particularly the purification of the envelope glycoproteins can be carried out as follows. This fixation can particularly be carried out by contacting a lysate of the LAV virus dissolved in a suitable buffer with concanavaline-A bound to SEPHAROSE®. A suitable buffer has the following composition :

| | |
|---|---|
| Tris | 10 mM |
| NaCl | 0.15 M |
| CaCl₂ | 1 mM |
| MgCl₂ | 1 mM |
| Detergent marketed under the trade mark TRITON 1 % | |
| pH | 7.4 |

When the fixation has been achieved, the SEPHAROSE-concanavaline A is washed with a buffer of the same composition, except that the TRITON® concentration is lowered to 0.1 %. The elution is then effected with an 0.2 M O-methyl-α-D-mannopyranoside solution in the washing buffer.

The protein may be further concentrated by immuno-precipitation with antibodies contained in the serums of patients infected with AIDS or with polyclonal antibodies obtained from a serum derived from an animal previously immunised against the "unaltered" virus according to the invention or the abovesaid glycoprotein. The protein can then be recovered by dissociation of the complex by a solution having an adequate content of ionic salt. Preferably the antibody preparation is itself immobilised in a manner known in itself on an insoluble support, for instance of the SEPHAROSE® B type.

It is also possible to resort to monoclonal antibodies secreted by hybridomas previously prepared against gp 110. These monoclonal antibodies, as well as the hybridomas which produce them, also form part of the invention.

A technique for producing and selecting monoclonal antibodies directed against the gp110 glycoprotein is described below.

### Immunisation of the mice

Groups of Balb/c mice from 6 to 8 weeks old mice were used. One group receives the virus carrying the abovesaid glycoprotein, another a purified glycoprotein gp110. The immunisation procedure, identical for all mice, comprises injecting 10 mg of the antigenic preparation in the presence of Freund complete adjuvant at day 0, then again but in the presence of Freund incomplete adjuvant at day 14 and without adjuvant at days 28 and 42. The three first injections are made intraperitoneally, the fourth intravenously.

### Fusion and culture of the hybrids

The non secreting myeloma variant 5.53 P3 x 63 Ag8, resistant to azaguanine, itself derived from the MOPC-21 cell-line, is used. Fusion with immunised mouse splenocytes is carried out in the presence of polyethylene-glycol 4000 by the technique of FAZEKAS de st-GROTH and SCHEIDEGGER on the 45th day. The selection of the hybrids in RPMI 16-40 "HAT" medium is carried out in plates having 24 cups (known under the designation COSTAR) by resorting to the same culture techniques.

The hybridomas producing antibodies of adequate specificity are then cloned in plates having 96 cups, in the presence of a "feeder" layer of syngenic thymocytes. The producing clones thus selected are then expanded in 24 cup plates, still in the presence of thymocytes. When the confluence appears in one of the cups, the clone is injected intraperitoneally into a balbic mouse which had received an injection of PRISTANE 8 days previously and/or kept in liquid culture.

### Demonstration of the anti-LAV antibodies

Five different techniques enable characterisation of the clones producing antibodies of suitable specificity. In a first stage, the hybrids producing antibodies are determined by an ELISA test revealing mouse immunoglobulins in the supernatant liquors. From this first selection, supernatants are sought which have antibodies directed against viral constituents by means of an ELISA test revealing anti-LAV antibodies, or by immunofluorescence on the virus producing human cells. Finally the supernatant liquours are analysed by radio-immunoprecipitation of virus labelled with cystein and by the Western-Blot technique on viral preparation which permit the determination of the specificities of these anti-LAV antibodies.

### RESULTS

Cells obtained from the various fusions are placed under culture in 648 cups. Their microscopic examination shows that the majority of these cups contain a single hybrid clone capable of growing in a "HAT" selective medium. More than 50 % among them produce antibodies giving rise to a positive response under ELISA antivirus examination. The most representative fusions are tested by the Western-Blot technique and several of them are subcloned, taking into account their respective specificities reactivities in antivirus ELISA and their behaviours under the culturing conditions. Those hybrids which are more particularly selected are those which produce antibodies which selectively recognise the viral glycoprotein gp110 having a molecular weight of about 110 KD. All the sub clonings give rise to clones producing antibodies which, after expression, are injected into syngenic mice. Analysis of the specificities of the antibodies present in the different ascites liquids confirm the specificity of the antibodies of said ascites with respect to gp110.

The monoclonal antibodies obtained can themselves be employed to purify proteins containing an antigenic site also contained in gp110. The invention relates therefore also to these processes of purification as such. This process is advantageously applied to virus lysates or T lymphocyte lysates or other cells producing LAV or the like, when care has been taken to avoid the uncontrolled separation of gp110 during the purification procedure of the virus, prior to lysys thereof. Needless to say that the process can also be applied to any solution containing gp110 or a protein, polypeptide or glycoprotein comprising an antigenic site normally carried by the envelope protein and recognised by the monoclonal antibody. For practising this process, the monoclonal antibodies are advantageously immobilised on a solid support, preferably adapted to affinity chromatography operations. For example, these monoclonal antibodies are fixed to an agarose lattice with three-dimensional cross-linking, marketed under the trade mark SEPHAROSE by the Swedish company PHARMACIA A.G., for example by the cyanogen bromide method.

The invention therefore also relates to a process for separating the antigens concerned, which process comprises contacting a mixture of antigens, including those of interest (for instance a virus lysate or extract), with an affinity column bearing the above-said monoclonal antibodies, to selectively fix polypeptides, proteins or glycoproteins selectively recognized by said monoclonal antibodies, recovering the latter by dissociation of the antigen-antibody complex by means of a suitable buffer, particularly a solution of adequate ionic strength, for example of a salt, preferably ammonium acetate (which leaves no residue upon freeze drying of the preparation or a solution acidified to a pH 2-4 or to a glycine buffer at the same pH and recovering the eluted polypeptides, proteins or glycoproteins.

It is self-evident that the invention relates also to polypeptide fragments having lower molecular weights and carrying antigenic sites recognizable by the same monoclonal antibodies. It is clear to the specialist that the availabililty of monoclonal antibodies recognizing the gp110 glycoprotein gives also access to smaller peptide sequences or fragments containing the common antigenic site or epitope. Fragments of smaller sizes may be obtained by resorting to known techniques. For instance such a method comprises cleaving the original larger polypeptide by enzymes capable of cleaving it at specific sites. By way of examples of such proteins, may be mentioned the enzyme of Staphylococcyus aureus V8, α-chymotrypsin, "mouse sub-maxillary gland protease" marketed by the BOEHRINGER company, Vibrio alginolyticus chemovar iophagus collagenase, which specifically recognises said peptides Gly-Pro and Gly-Ala, etc..

It is also possible to obtain polypeptides or fragments of envelope antigens of the virus, by cloning fragments excised from a cDNA constructed from genoimes of LAV variants.

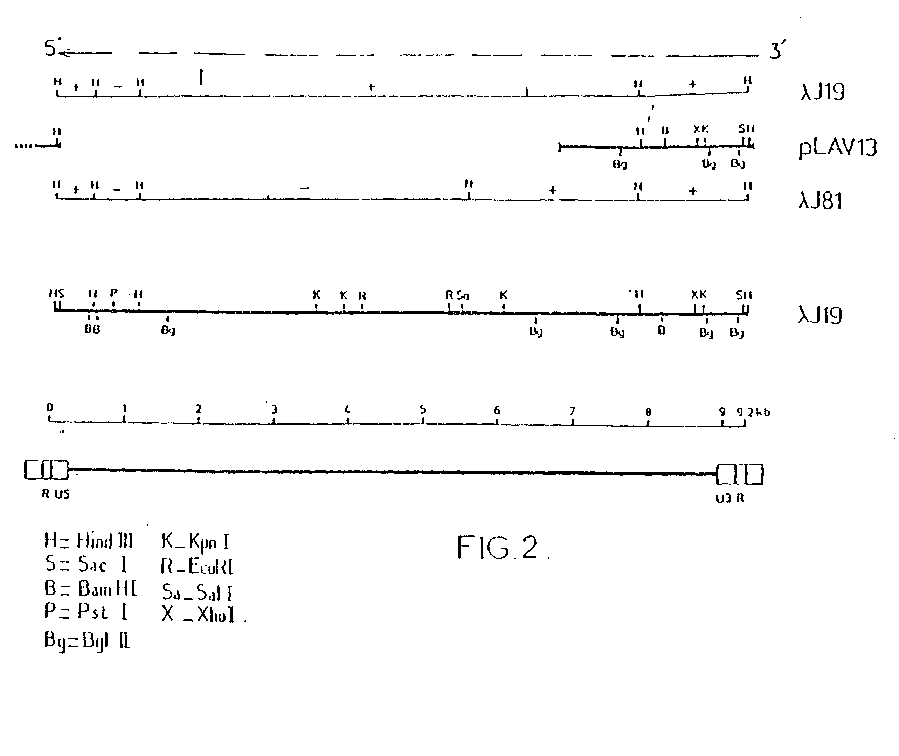

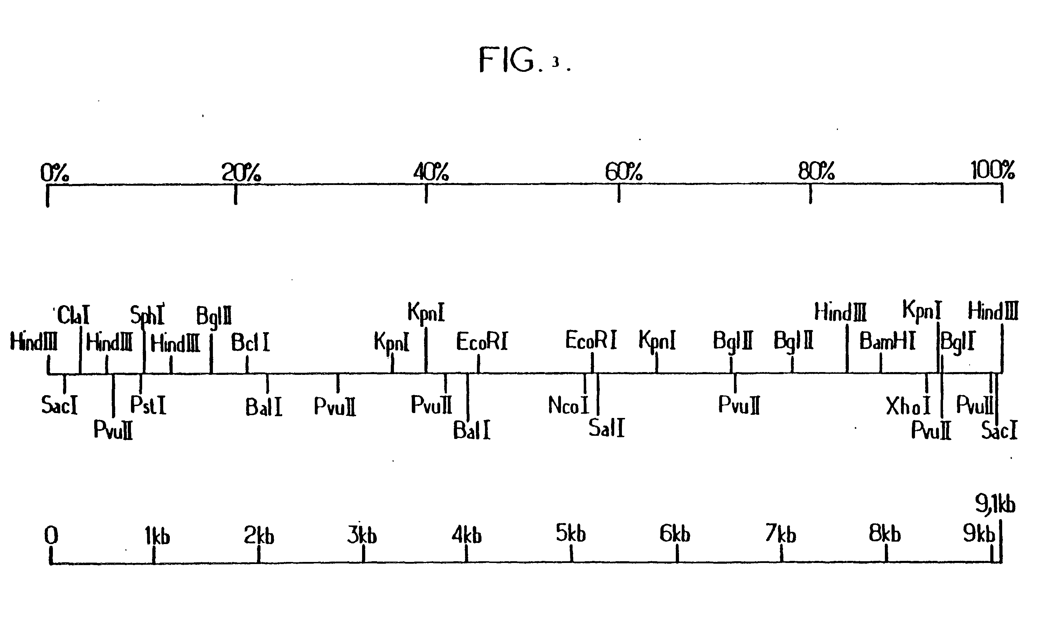

Figures 2 and 3 are restriction maps of such a cDNA comprising a total of 9.1 to 9.2 kb. The polypeptides coded by cDNA fragments located in the region extending between site Kpnl (position 6100) and site BgIII (position 9150) of the restriction map of Figure 2. The presence of a characteristic site of an envelope antigen of the LAV virus or the like in any polypeptide expressed (in a suitable host cell transformed behorehand by a corresponding fragment or by a vector containing said fragment) can be detected by any suitable immunochemical means.

Particularly the invention relates more particularly to polypeptides encoded by cDNA fragments defined hereafter. It also relates to the nucleic acid fragments themselves, including a cDNA variant corresponding to a whole LAV retroviral genome, characterized by a series of restriction sites in the order hereafter (from the 5' end to the 3' end).

The coordinates of the successive sites of the whole LAV genome (see also restriction map of λJ19 in fig. 2) are indicated hereafter too, with respect to the Hind III site (selected as of coordinate 1) which is located in the R region. The coordinates are estimated with an accuracy of ± 200 bp :

| | |
|---|---|
| Hind III | 0 |
| Sac I | 50 |
| Hind III | 520 |
| Pst I | 800 |
| | |
| Hind III | 1 100 |
| Bgl II | 1 500 |
| Kpn I | 3 500 |
| Kpn I | 3 900 |
| Eco RI | 4 100 |
| Eco RI | 5 300 |
| Sal I | 5 500 |
| Kpn I | 6 100 |
| Bgl II | 6 500 |
| Bgl II | 7 600 |
| Hind III | 7 850 |
| Bam HI | 8 150 |
| Xho I | 8 600 |
| Kpn I | 8 700 |
| Bgl II | 8 750 |
| Bgl II | 9 150 |
| Sac I | 9 200 |
| Hind III | 9 250 |

Another DNA variant according to this invention optionally contains an additional Hind III approximately at the 5 550 coordinate.

Reference is further made to fig. 1 which shows a more detailed restriction map of said whole-DNA (λJ19).

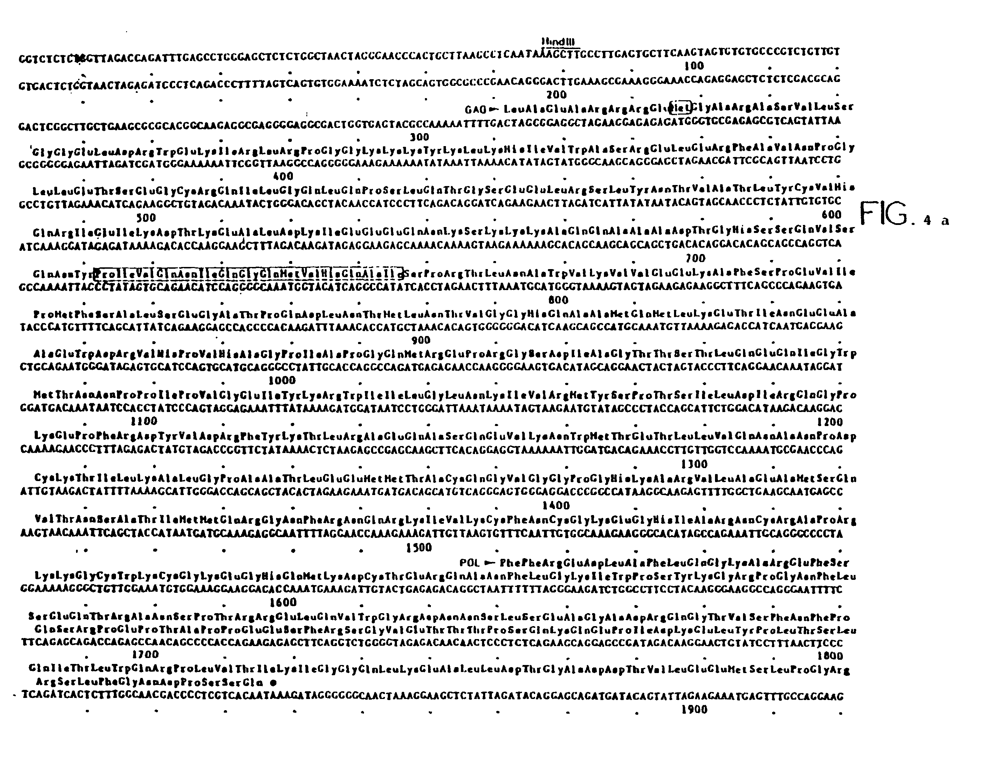

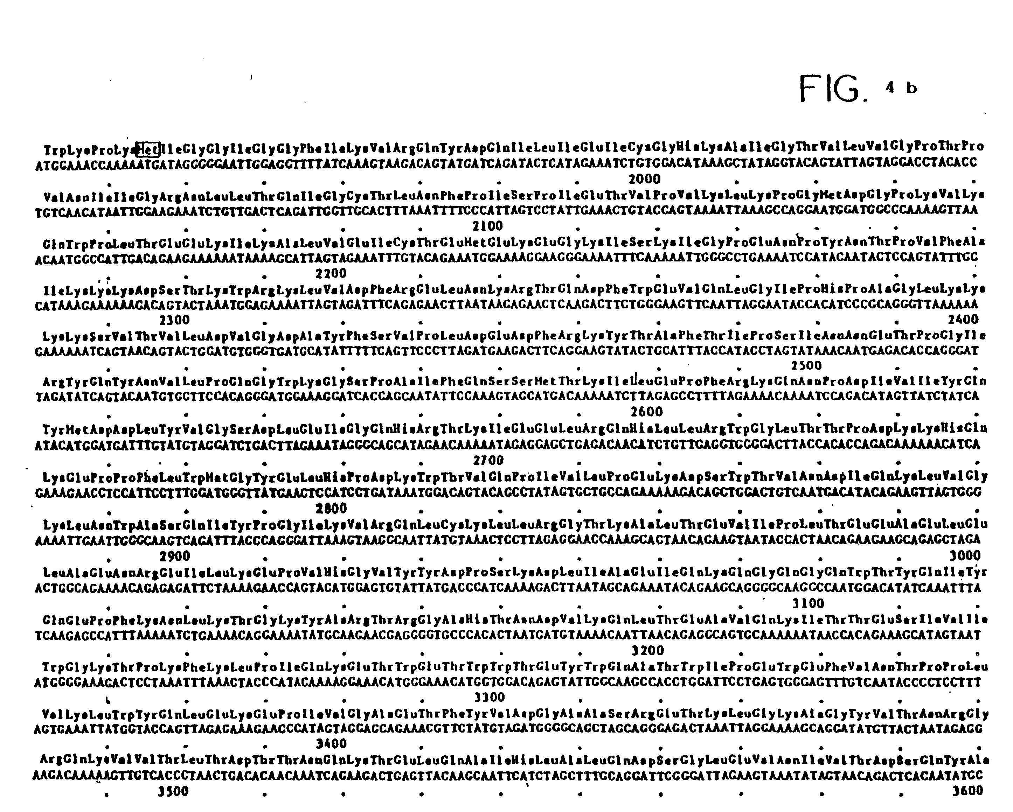

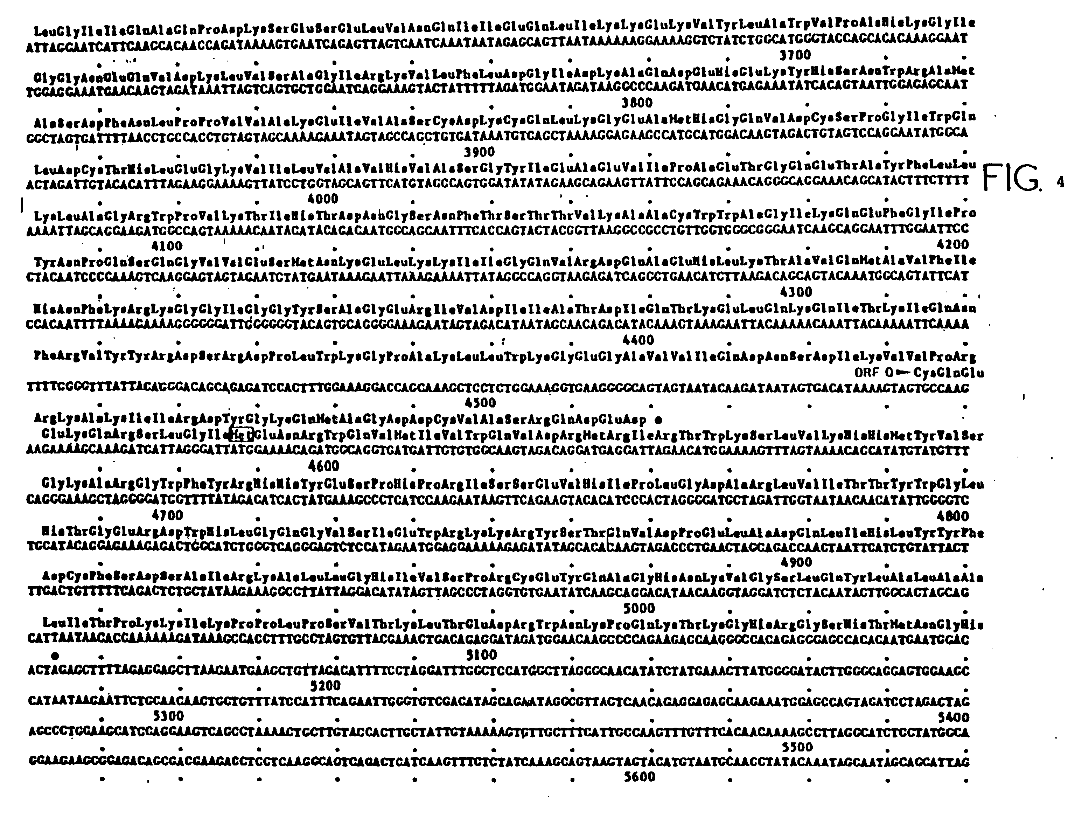

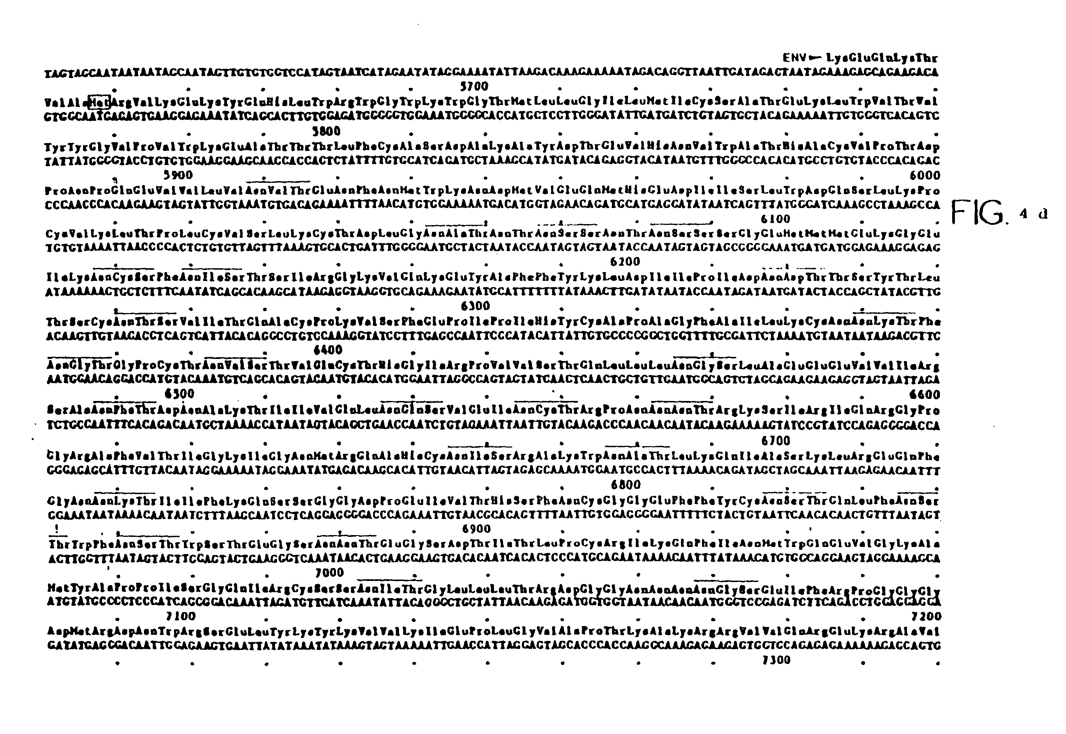

An even more detailed nucleotidic sequence of a preferred DNA according to the invention is shown in figs. 4a-4e hereafter.

The invention further relates to other preferred DNA fragments and polypeptide sequences (glycosylated or not glycosylated) which will be referred to hereafter.

### SEQUENCING OF LAV

The sequencing and determination of sites of particular interest were carried out on a phage recombinant corresponding to λJ19 disclosed in the abovesaid EP-A-0178978. A method for preparing it is disclosed in that application.

The whole recombinant phage DNA of clone λJ19 (disclosed in the earlier application) was sonicated according to the protocol of DEININGER (1993), Analytical Biochem. 129, 216. the DNA was repaired by a Klenow reaction for 12 hours at 16° C. The DNA was electrophoresed through 0.8 % agarose gel and DNA in the size range of 300-600 bp was cut out and electroeluted and precipitated. Resuspended DNA (in 10 mM Tris, pH 8 ; 0,1 mM EDTA) was ligated into M13mp8 RF DNA (cut by the restriction enzyme Smal and subsequently alkaline phosphated), using T4 DNA-and RNA-ligases (Maniatis T et al (1992) - Molecular cloning - Cold Spring Harbor Laboratory). An E. coli strain designated as TG1 was used for further study. This strain has the following genotype :
Δlac pro, supE, thi.F'traD36, proAB, lacl^{q}, ZΔM15,r⁻
This E. coli TGI strain has the peculiarity of enabling recombinants to be recognized easily. The blue colour of the cells transfected with plasmids which did not recombine with a fragment of LAV DNA is not modified. To the contrary cells transfected by a recombinant plasmid containing a LAV DNA fragment yield white colonies. The technique which was used is disclosed in Gene (1983), 26, 101.

This strain was transformed with the ligation mix using the Hanahan method (Hanahan D (1983) J. Mol. Biol. 166, 557). Cells were plated out on tryptone-agarose plate with IPTG and X-gal in soft agarose. White plaques were either picked and screened or screened directly in situ using nitrocellulose filters. Their DNAs were hybridized with nick-translated DNA inserts of pUC18 Hind III subclones of λJ19. This permitted the isolation of the plasmids or subclones of λ which are identified in the table hereafter. In relation to this table it should also be noted that the designation of each plasmid is followed by the deposition number of a cell culture of E. coli TGI containing the corresponding plasmid at the "Collection Nationale des Cultures de Micro-organismes" (C.N.C.M.) of the Pasteur Institute in Paris, France. A non-transformed TGI cell line was also deposited at the C.N.C.M. under Nr. 1-364. All these deposits took place on November 15, 1984. The sizes of the corresponding inserts derived from the LAV genome have also been indicated.

**TABLE**

| Essential features of the recombinant plasmids | |
|---|---|
| - pJ19 - 1 plasmid (I-365) | 0.5 kb |
| | |
| Hind III - Sac I - Hind III | |
| | |
| - pJ19 - 17 plasmid (I-367) | 0.6 kb |
| | |
| Hind III - Pst 1 - Hind III | |
| | |
| - pJ19 - 6 plasmid (I-366) | 1.5 kb |
| | |
| Hind III (5') | |
| Bam HI | |
| Xho I | |
| Kpn I | |
| Bgl II | |
| Sac I (3') | |
| Hind III | |
| | |
| - pJ19-13 plasmid (I-368) | 6.7 kb |
| | |
| Hind III (5') | |
| Bgl II | |
| Kpn I | |
| Kpn I | |
| Eco RI | |
| Eco RI | |
| Sal I | |
| Kpn I | |
| Bgl II | |
| Bgl II | |
| Hind III (3') | |

Positively hybridizing M13 phage plates were grown up for 5 hours and the single-stranded DNAs were extracted.

M13mp8 subclones of λJ19 DNAs were sequenced according to the dideoxy method and technology devised by Sanger et al (Sanger et al (1977), Proc. Natl. Acad. Sci. USA, 74 , 5463 and M13 cloning and sequencing handbook, AMERSHAM (1983). the 17-mer oligonucleotide primer α-³⁵ SdATP (400Ci/mmol, AMERSHAM), and 0.5X-5X buffer gradient gels (Biggen M.D. et al (1983, Proc. Natl. Acad. Sci. USA, 50, 3963) were used. Gels were read and put into the computer under the programs of Staden (Staden R. (1982), Nucl. Acids Res. 10, 4731). All the appropriate references and methods can be found in the AMERSHAM M13 cloning and sequencing handbook.

The complete DNA sequence of λJ19 (also designated as LAV-la) is shown in figs. 4 to 4e.

The sequence was reconstructed from the sequence of phage λJ19 insert. The numbering starts at the cap site which was located experimentally (see hereafter). Important genetic elements, major open reading frames and their predicted products are indicated together with the HindIII cloning sites. The potential glycosylation sites in the env gene are overlined. The NH₂-terminal sequence of p25^{gag} determined by protein microsequencing is boxed.

Each nucleotide was sequenced on average 5.3 times : 85 % of the sequence was determined on both strands and the remainder sequenced at least twice from independent clones. The base composition is T, 22.2 % ; C, 17.8 % ; A, 35.8 % ; G, 244.2 % ; G + C, 42 %. The dinucleotide GC is greatly under represented (0,9 %) as common amongst eukaryotic sequences (Bird 1980).

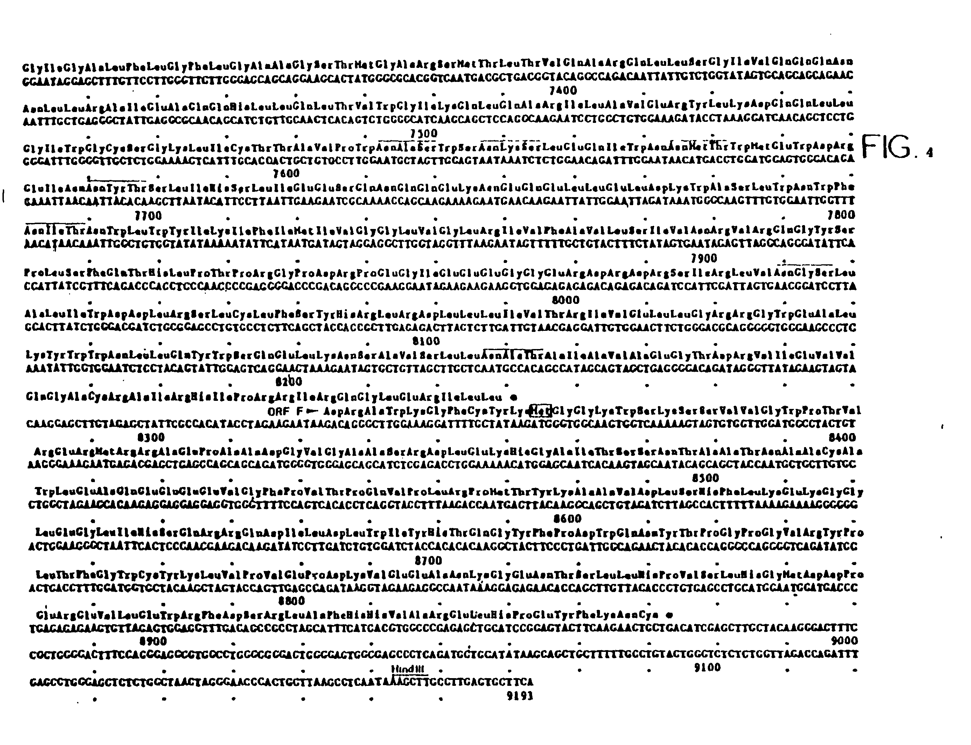

Figs. 5 and 6 provide a diagrammatized representation of the lengths of the successive open reading frames corresponding to the successive reading phases (also referred to by numbers "1", "2" and "3" appearing in the left handside part of fig. 5). The relative positions of these open reading frames (ORF) with respect to the nucteotidic structure of the LAV genome is referred to by the scale of numbers representative of the respective positions of the corresponding nucleotides in the DNA sequence. The vertical bars correspond to the positions of the corresponding stop codons.

The following genes and DNA fragments can be distinguished on the different reading frames shown.

The env gene and other genes are also referred to in the table hereafter.

Figs. 4a-4c also show that the DNA fragment extending from nucleotidic position 1631 (starting with 5'TTT TTT ....3' to nucleotidic position 5162 thought to correspond to the pol gene. The polypeptidic structure of the corresponding polypeptides is deemed to be that corresponding to phase 1. It stops at position 4639 (end by 5'G GAT GAG GAT 3').

These genes are thought to code for the virus polymerase or reverse transcriptase.

### 3) The envelope gene (or ORF-env)

env : The env open reading frame has a possible initiator methionine codon very near the beginning (8th triplet). If so the molecular weight of the presumed env precursor protein (861 aminoacids, MWcalc = 97376) is consistent with the size of the LAV glycoprotein (110 kd and 90 kd after glycosidase treatment). There are 32 potential N-glycosylation sites (Asn-X-Ser/Thr) which are overlined in Fig. 4d and 4e. An interesting feature of env is the very high number of Trp residues at both ends of the protein.

The DNA sequence thought to code for envelope proteins is thought to extend from nucleotidic position 5746 (starting with 5' AAA GAG GAG A....3') up to nucleotidic position 8208 (ending by ....A ACT AAA GAA 3'). Polypeptidic structures of sequences of the envelope protein correspond to those read according to the "phase 3" reading phase.

The start of env transcription is thought to be at the level of the ATG codon at position 5767-5769.

There are three hydrophobic regions, characteristic of the retroviral envelope proteins (Seiki et al., 1983) corresponding to a signal peptide (encoded by nucleotides 5815-5850 bp), a second region (7315-7350 bp) and a transmembrane segment (7831-7890 bp). The second hydrophobic region (7315-7350 bp) is preceeded by a stretch rich in Arg + Lys. It is possible that this represents a site of proteolytic cleavage, which by analogy with over retroviral proteins, would give an external envelope polypeptide and a membrane associated protein (Seiki et al., 1983, Kiyokawa et al., 1984). A striking feature of the LAV envelope protein sequence is that the segment following the transmembrane segment is of unusual length (150 residues). The env protein shows no homology to any sequence in protein data banks. The small aminoacid motif common to the transmembrane proteins of all leukemogenic retroviruses (Cianciolo et al., 1984) is not present in lav env.

The invention concerns more particularly the DNA sequence extending from nucleotide 5767 up to nucleotide 7314 deemed to encode the gp 110 (envelope glycoprotein of the LAV virus which has a molecular weight of about 110,000 daltons) beginning at about nucleotide as well as the polypeptidic backbone of the glycoprotein sequence wich corresponds to that having an approximate molecular weight which was initially believed to be 90,000 daltons, and which turned out to be 55,000. The polypeptide resulting from the complete removal of sugar residues of gp110 can be obtained by the treatment of said gp 110 with the appropriate glycosidase.

The invention concerns more particularly the DNA sequences extending from nucleotides 567, up to nucleotide 8208 or 8350 deemed to encode the gp 110 (envelope glycoprotein of the LAV virus which has a molecular weight of about 110,000 daltons) beginning at about nucleotide as well as the polypeptidic backbone of the glycoprotein sequence which corresponds to that having an approximate molecular weight of 90,000 daltons and which is obtained by glycosidase treatment of gp110.

The invention further relates to the purified polypeptides which have the aminoacid structure (or polypeptidic backbone) of the gp110 and gp90 respectively, which correspond to the direct translation of the DNA sequences and fragments which have been defined more specifically hereabove (figs 4d and 4e).

The invention further relates to polypeptides containing neutralizing epitopes.

The locations of neutralizing epitopes are further apparent in fig. 4d. Reference is more particularly made to the overlined groups of three letters included in the aminoacid sequences of the envelope proteins (reading phase 3) which can be designated generally by the formula Asn-X-Ser or Asn-X-Thr, wherein X is any other possible aminoacid. Thus the initial protein product or polypeptide backbone of the env glycoprotein has a molecular weight in excess of 91,000. These groups are deemed to generally carry glycosylated groups. These Asn-X-Ser and Asn-X-Thr groups with attached glycosylated groups form together hydrophylic regions of the protein and are deemed to be located at the periphery of and to be exposed outwardly with respect to the normal conformation of the proteins. Consequently they are considered as being epitopes which can efficiently be brought into play in vaccine compositions.

Other hydrophilic peptides in the env open reading frame are identified hereafter. they are defined starting from aminoacid 1 = lysine coded by the AAA at position 5746-5748 in the LAV DNA sequence (figs 4d and 4e) and then further numbered in accordance with their order with respect to the end sequence. The first and second numbers in relation to each peptide refer to their respective N-terminal and C-terminal aminoacids.

These hydrophilic peptides are :

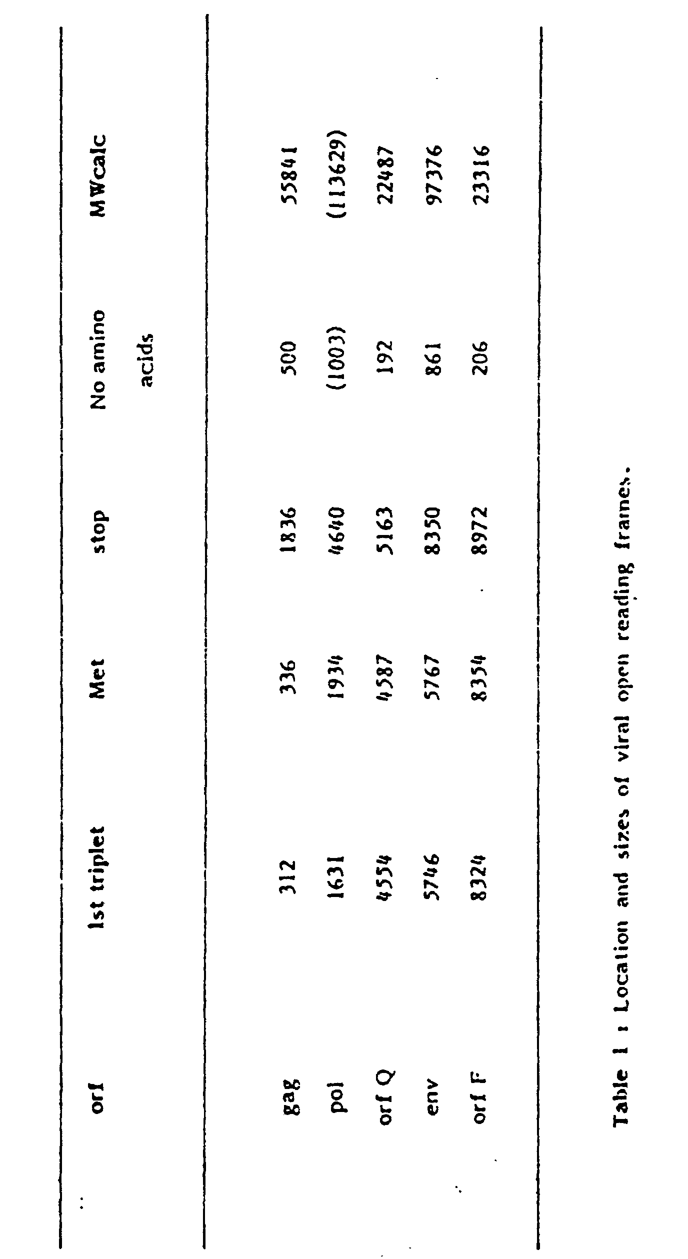

aminoacids 8-23 inclusive, i.e. Met-Arg-Val-Lys-Glu-Lys-
Tyr-Gln-His-Leu-Trp-Arg-Trp-Gly-Trp-Lys-
aminoacids 63-78 inclusive, i.e. Ser-Asp-Ala-Lys-Ala-Tyr-
Asp-Thr-Glu-Val-His-Asn-Val-Trp-Ala-Thr-
aminoacids 82-90 inclusive, i.e. Val-Pro-Thr-Asp-Pro-Asn-
Pro-Gln-Glu-

aminoacids 97-123 inclusive, i.e. Thr-Glu-Asn-Phe-Asn-
Met-Trp-Lys-Asn-Asp-Met-Val-Glu-Gln-Met-His-Glu-
Asp-Ile-Ile-Ser-Leu-Trp-Asp-Gln-Ser-Leu-
aminocids 127-183 inclusive, i.e. Val-Lys-Leu-Thr-Pro-
Leu-Cys-Val-Ser-Leu-Lys-Cys-Thr-Asp-Leu-Gly-Asn-
Ala-Thr-Asn-Thr-Asn-Ser-Ser-Asn-Thr-Asn-Ser-Ser-
Ser-Gly-Glu-Met-Met-Met-Glu-Lys-Gly-Glu-Ile-Lys-
Asn-Cys-Ser-Phe-Asn-Ile-Ser-Thr-Ser-Ile-Arg-Gly-
Lys-Val-Gln-Lys-
aminoacids 197-201 inclusive, i.e. Leu-Asp-Ile-Ile-Pro-
Ile-Asp-Asn-Asp-Thr-Thr-
aminocids 239-294 inclusive, i.e. Lys-Cys-Asn-Asn-Lys-
Thr-Phe-Asn-Gly-Thr-Gly-Pro-Cys-Thr-Asn-Val-Ser-
Thr-Val-Gln-Cys-Thr-His-Gly-Ile-Arg-Pro-Val-Val-
Ser-Thr-Gln-Leu-Leu-Leu-Asn-Gly-Ser-Leu-Ala-Glu-
Glu-Glu-Val-Val-Ile-Arg-Ser-Ala-Asn-Phe-Thr-Asp-
Asn-Ala-Lys-
aminocids 300-327 inclusive,i.e. Leu-Asn-Gln-Ser-Val-Glu-
Ile-Asn-Cys-Thr-Arg-Pro-Asn-Asn-Asn-Thr-Arg-Lys-
Ser-Ile-Arg-Ile-Gln-Arg-Gly-Pro-Gly-Arg-
aminoacids 334-381 inclusive, i.e. Lys-Ile-Gly-Asn-Met-
Arg-Gln-Ala-His-Cys-Asn-Ile-Ser-Arg-Ala-Lys-Trp-
Asn-Ala-Thr-Leu-Lys-Gln-Ile-Ala-Ser-Lys-Leu-Arg-
Glu-Gln-Phe-Gly-Asn-Asn-Lys-Thr-Ile-Ile-Phe-Lys-
Gln-Ser-Ser-Gly-Gly-Asp-Pro-
aminoacids 397-424 inclusive, i.e. Cys-Asn-Ser-Thr-Gln-
Leu-Phe-Asn-Ser-Thr-Trp-Phe-Asn-Ser-Thr-Trp-Ser-
Thr-Glu-Gly-Ser-Asn-Asn-Thr-Glu-Gly-Ser-Asp-
aminoacids 466-500 inclusive, i.e. Leu-Thr-Arg-Asp-Gly-
Gly-Asn-Asn-Asn-Asn-Gly-Ser-Glu-Ile-Phe-Arg-Pro-
Gly-Gly-Gly-Asp-Met-Arg-Asp-Asn-Trp-Arg-Ser-Glu-
Leu-Tyr-Lys-Tyr-Lys-Val-
aminoacids 510-523 inclusive, i.e. Pro-Thr-Lys-Ala-Lys-
Arg-Arg-Val-Val-Gln-Arg-Glu-Lys-Arg-

aminoacids 551-577 inclusive, i.e. Val-Gln-Ala-Arg-Gln-
Leu-Leu-Ser-Gly-Ile-Val-Gln-Gln-Gln-Asn-Asn-Leu-
Leu-Arg-Ala-Ile-Glu-Ala-Gln-Gln-His-Leu-
aminoacids 594-603 inclusive, i.e. Ala-Val-Glu-Arg-Tyr-
Leu-Lys-Asp-Gln-Gln-
aminoacids 621-630 inclusive, i.e. Pro-Trp-Asn-Ala-Ser-
Trp-Ser-Asn-Lys-Ser-
aminoacids 657-679 inclusive, i.e. Leu-Ile-Glu-Glu-Ser-
Gln-Asn-Gln-Gln-Glu-Lys-Asn-Glu-Gln-Glu-Leu-Leu-
Glu-Leu-Asp-Lys-Trp-Ala-
aminoacids 719-758 inclusive, i.e. Arg-Val-Arg-Gln-Gly-
Tyr-Ser-Pro-Leu-Ser-Phe-Gln-Thr-His-Leu-Pro-Thr-
Pro-Arg-Gly-Pro-Asp-Arg-Pro-Glu-Gly-Ile-Glu-Glu-
Glu-Gly-Gly-Glu-Arg-Asp-Arg-Asp-Arg-Ser-Ile-
aminoacids 780-803 inclusive, i.e. Tyr-His-Arg-Leu-Arg-
Asp-Leu-Leu-Leu-Ile-Val-Thr-Arg-Ile-Val-Glu-Leu-
Leu-Gly-Arg-Arg-Gly-Trp-Glu-

The invention concerns more particularly all the DNA fragments which have been more specifically referred to hereabove and which correspond to open reading frames. It will be understood that the man skilled in the art will be able to obtain them all, for instance by cleaving an entire DNA corresponding to the complete genome of a LAV species, such as by cleavage by a partial or complete digestion thereof with a suitable restriction enzyme and by the subsequent recovery of the relevant fragments. The different DNAs disclosed above can be resorted to also as a source of suitable fragments. The techniques disclosed hereafter for the isolation of the fragments which were then included in the plasmids referred to hereabove and which were then used for the DNA sequencing can be used.

Of course other methods can be used. Some of them have been examplified in EP-A-0178978.

Reference is for instance made to the following methods.
a) DNA can be transfected into mammalian cells with appropriate selection markers by a variety of techniques, calcium phosphate precipitation, polyethylene glycol, protoplast-fusion, etc..
b) DNA fragments corresponding to genes can be cloned into expression vectors for E. coli, yeast- or mammalian cells and the resultant proteins purified.
c) The provival DNA can be "shot-gunned" (fragmented) into procaryotic expression vectors to generate fusion polypeptides. Recombinant producing antigenically competent fusion proteins can be identified by simply screening the recombinants with antibodies against LAV antigens .

The invention further refers more specifically to DNA recombinants, particularly modified vectors, including any of the preceding DNA sequences and adapted to transform corresponding microorganisms or cells, particularly eucaryotic cells such as yeasts, for instance saccharomyces cerevisiae, or higher eucaryotic cells, particularly cells of mammals, and to permit expression of said DNA sequences in the corresponding microorganisms or cells.

More particularly the invention relates to such modified DNA recombinant vectors modified by the abovesaid DNA sequences and which are capable of transforming higher eucaryotic cells particularly mammalian cells. Preferably any of the abovesaid sequences are placed under the direct control of a promoter contained in said vectors and which is recognized by the polymerases of said cells, such that the first nucleotide codons expressed correspond to the first triplets of the above-defined DNA-sequences. Accordingly this invention also relates to the corresponding DNA fragments which can be obtained from LAV genomas or corresponding cDNAs by any appropriate method. For instance such a method comprises cleaving said LAV genomas or cDNAs by restriction enzymes preferably at the level of restriction sites surrounding said fragments and close to the opposite extremities respectively thereof, recovering and identifying the fragments sought according to sizes, if need be checking their restriction maps or nucleotide sequences (or by reaction with monoclonal antibodies specifically directed against epitopes carried by the polypeptides encoded by said DNA fragments), and further if need be, trimming the extremities of the fragment, for instance by an exonucleolytic enzyme such as Bal31, for the purpose of controlling the desired nucleotide-sequences of the extremities of said DNA fragments or, conversely, repairing said extremities with Klenow enzyme and possibly ligating the latter to synthetic polynucleotide fragments designed to permit the reconstitution of the nucleotide extremities of said fragments. Those fragments may then be inserted in any of said vectors for causing the expression of the corresponding polypeptide by the cell transformed therewith. The corresponding polypeptide can then be recovered from the transformed cells, if need be after lysis thereof, and purified, by methods such as electrophoresis. Needless to say that all conventionnal methods for performing these operations can be resorted to.

The invention also relates more specifically to cloned probes which can be made starting from any DNA fragment according to this invention, thus to recombinant DNAs containing such fragments, particularly any plasmids amplifiable in procaryotic or eucaryotic cells and carrying said fragments.

Using the cloned DNA fragments as a molecular hybridization probe - either by labelling with radionucleotides or with fluorescent reagents - LAV virion RNA may be detected directly in the blood, body fluids and blood products (e.g. of the antihemophylic factors such as Factor VIII concentrates) and vaccines, i.e. hepatitis B vaccine. It has already been shown that whole virus can be detected in culture supernatants of LAV producing cells. A suitable method for achieving that detection comprises immobilizing virus onto a support, e.g. nitrocellulose filters, etc., disrupting the virion and hybridizing with labelled (radiolabelled or "cold" fluorescent- or enzyme-labelled) probes. Such an approach has already been developed for Hepatitis B virus in peripheral blood (according to SCOTTO J. et al. Hepatology (1983), 3, 379-384).

Probes according to the invention can also be used for rapid screening of genomic DNA derived from the tissue of patients with LAV related symptoms, to see if the proviral DNA or RNA is present in host tissue and other tissues.

A method which can be used for such screening comprises the following steps : extraction of DNA from tissue, restriction enzyme cleavage of said DNA, electrophoresis of the fragments and Southern blotting of genomic DNA from tissues, subsequent hybridization with labelled cloned LAV provival DNA. Hybridization in situ can also be used.

Lymphatic fluids and tissues and other non-lymphatic tissues of humans, primates and other mammalian species can also be screened to see if other evolutionnary related retrovirus exist. The methods referred to hereabove can be used, although hybridization and washings would be done under non stringent conditions.

The DNAs or DNA fragments according to the invention can be used also for achieving the expression of LAV viral antigens for diagnostic purposes.

The invention relates generally to the polypeptides themselves, whether synthetized chemically isolated from viral preparation or expressed by the different DNAs of the inventions, particularly by the ORFs or fragments thereof, in appropriate hosts, particularly procaryotic or eucaryotic hosts, after transformation thereof with a suitable vector previously modified by the corresonding DNAs.

More generally, the invention also relates to any of the polypeptide fragments (or molecules, particularly glycoproteins having the same polypeptidic backbone as the polypeptides mentioned hereabove) bearing an epitope characteristic of a LAV protein or glycoprotein, which polypeptide or molecule then has N-terminal and C-terminal extremities respectively either free or, independently from each other, covalently bond to aminoacids other than those which are normally associated with them in the larger polypeptides or glycoproteins of the LAV virus, which last mentioned aminoacids are then free or belong to another polypeptidic sequence. Particularly the invention relates to hybrid polypeptides containing any of the epitope-bearing-polypeptides which have been defined more specifically hereabove, recombined with other polypeptides fragments normally foreign to the LAV proteins, having sizes sufficient to provide for an increased immunogenicity of the epitope-bearing-polypeptide yet, said foreign polypeptide fragments either being immunogenically inert or not interfering with the immunogenic properties of the epitope-bearing-polypeptide.

The glycoproteins, proteins and polypeptides (generally designated hereafter as "antigens" of this invention, whether obtained in a purified state from LAV virus preparations or by DNA-recombinant technology, are useful in processes for the detection of the presence of anti-LAV antibodies in biological media, particularly biological fluids such as sera from man or animal, particularly with a view of possibly diagnosing LAS or AIDS.

Particularly the invention relates to an in vitro process of diagnosis making use of an envelope glycoprotein (or of a polypeptide bearing an epitope of this glycoprotein) for the detection of anti-LAV antibodies in the serums of persons who carry them. Other polypeptides - particular those carrying an epitope of a core protein - can be used too.

A preferred embodiment of the process of the invention comprises :
- depositing a predetermined amount of one or several of said antigens in the cups of a titration miroplate :

- introducing of increasing dilutions of the biological fluid, i.e. serum to be diagnosed into these cups :

- incubating the microplate :

- washing carefully the microplate with an appropriate buffer:

- adding into the cups specific labelled antibodies directed against blood immunoglobulins and
- detecting the antigen-antibody-complex formed, which is then indicative of the presence of LAV antibodies in the biological fluid.

Advantageously the labelling of the anti-immunoglobulin antibodies is achieved by an enzyme selected from among those which are capable of hydrolysing a substrate, which substrate undergoes a modification of its radiation-absorption, at least within a predetermined band of wavelenghts. The detection of the substrate, preferably comparatively with respect to a control, then provides a measurement of the potential risks or of the effective presence of the disease.

Thus preferred methods immuno-enzymatic or also immunofluorescent detections, in particular according to the ELISA technique. Titrations may be determinations by immunofluorescence or direct or indirect immunoenzymatic determinations. Quantitative titrations of antibodies on the serums studied can be made.

The invention also relates to the diagnostic kits themselves for the in vitro detection of antibodies against the LAV virus, which kits comprise any of the polypeptides identified herein, and all the biological and chemical reagents, as well as equipment, necessary for peforming diagnostic assays. Preferred kits comprise all reagents required for carrying out ELISA assays. Thus preferred kits will include, in addition to any of said polypeptides, suitable buffers and anti-human immunoglobulins, which anti-human immunoglobulins are labelled either by an immunofluorescent molecule or by an enzyme. In the last instance preferred kits then also comprise a substrate hydrolysable by the enzyme and providing a signal, particularly modified absorption of a radiation, at least in a determined wavelength, which signal is then indicative of the presence of antibody in the biological fluid to be assayed with said kit.

The invention also relates to vaccine compositions whose active principle is to be constituted by any of the antigens, i.e. the hereabove disclosed polypeptides whole antigens, particularly the purified gp110 or immunogenic fragments thereof, fusion polypeptides or oligopeptides in association with a suitable pharmaceutical or physiologically acceptable carrier.

A preferred active principle is the gp110 immunogen.

By way of example having no limitative character, there may be mentioned that suitable dosages of the vaccine compositions are those which are effective to elicit antibodies in vivo, in the host, particularly a human host. Suitable doses range from 10 to 500 micrograms of polypeptide, protein or glycoprotein per kg, for instance 50 to 100 micrograms per kg.

The different peptides according to this invention can also be used themselves for the production of antibodies, preferably monoclonal antibodies specific of the different peptides respectively. For the production of hybridomas secreting said monoclonal antibodies, conventional production and screening methods are used. These monoclonal antibodies, which themselves are part of the invention then provide very useful tools for the identification and even determination of relative proportions of the different polypeptides or proteins in biological samples, particularly human samples containing LAV or related viruses.

The invention further relates to the hosts (procaryotic or eucaryotic cells) which are transformed by the above mentioned recombinants and which are capable of expressing said DNA fragments.

Finally the invention also concerns vectors for the transformation of eucaryotic cells of human origin. particularly lymphocytes, the polymerases of which are capable of recognizing the LTRs of LAV. Particularly said vectors are characterized by the presence of a LAV LTR therein, said LTR being then active as a promoter enabling the efficient transcription and translation in a suitable host of a DNA insert coding for a determined protein placed under its controls.

Needless to say that the invention extends to all variants of genomes and corresponding DNA fragments (ORFs) having substantially equivalent properties, all of said genomes belonging to retroviruses which can be considered as equivalents of LAV.

It must be understood that the claims which follow are also intended to cover all equivalents of the products (glycoproteins, polypeptides, DNAs, etc..), whereby an equivalent is a product, i.e. a polypeptide which may distinguish from a determined one defined in any of said claims, say through one or several aminoacids, while still having substantially the same immunological or immunogenic properties. A similar rule of equivalency shall apply to the DNAs, it being understood that the rule of equivalency will then be tied to the rule of equivalency pertaining to the polypeptides which they encode.

It will also be understood that all the litterature referred to hereinbefore or hereinafter, and all patent applications or patents not specifically identified herein but which form counterparts of those specifically designated herein must be considered as incorporated herein by reference.

### REFERENCES

Alizon, M., Sonigo, P., Barre-Sinoussi, F., Chermann, J.C., Tiollais, P., Montagnier, L. and Wain-Hobson, S. (1984). Molecular cloning of lymphadenopathy-associated virus. Nature, in press.

Arya, S.K., Gallo, R.C., Hahn, B.H., Shaw, G.M., Popovic, M., Salahuddin, S.Z. and Wong-Staal, F. (1984). Homology of genome of AIDS-associated virus with genomes of human T-cell leukemia lymphoma viruses. Science 225, 927-930.

Barré-Sinoussi, F., Chermann, J.C., Rey, F., Nugeybe, M.T., Chamaret, S., Gruest, J., Dauguet, C.. Axler-Blin, C., Vezinet-Brun F., Rouzioux, C., Rozenbaum, W. and Montagnier, L. (1983). Isolation of a T-lymphotropic retrovirus from a patient at risk of Acquired Immune Deficiency Syndrome (AIDS). Science 220, 868-870.

Biggen, M.D., Gibson, T.J. and Hong, G.F. (1983). Buffer gradient gels and ³⁵S label as an aid to rapid DNA sequence determination. Proc. Natl. Acad. Sci. USA 80, 3963-3965.

Bird, A.P. (1980). DNA methylation and the frequency of CpG in animal DNA. Nucl. Acids Res. 8, 1499-1504.

Brun-Vézinet, F., Rouzioux, C., Barre-Sinoussi, F., Klatzmann, D., Saimot, A.G., Rozembaum, W., Montagnier, L. and Chermann, J.C. (1984). Detection of IgG antibodies to lymphadenopathy associated virus (LAV) by ELISA in patients with acquired immuno-deficiency syndrome of lymphadenopathy syndrome. Lancet I, 1253-1256.

Chen, H.R. and Barker, W.C. (1984). Nucleotide sequences of the retroviral long terminal repeats and their adjacent regions. Nucl. Acids Res. 12, 1767-1778.

Chen, I.S.Y., Mc Laughlin, J., Gasson, J.C., Clark, S.C. and Golde, D.W. (1983). Molecular characterization of genome of a novel human T-cell leukaemia virus. Nature 305, 502-505.

Chiu, I.M., Callahan, R., Tronick, S.R., Scholm, J. and Aaronson, S.A. (1984). Major pol gene progenitors in the evolution of oncornaviruses. Science 223, 364-370.

Cianciolo, G.J., Kipnis, R.J. and Snyderman, R. (1984). Similarity between p15E of murine and feline viruses and p21 of HTLV. Nature 311, 515.

Daly, H.M. and Scott, G.L. (1983). Fatal AIDS in a haemophiliae in the U.K. Lancet II, 1190.

Dittmar, K.J. and Moelling, K. (1978). Biochemical properties of p15-associated protease in an avion RNA tumor virus. J. Virol. 28, 106-118.

Donehower, L.A., Huang, A.L. and Hager, G.L. (1981). Regulatory and coding potential of the mouse mammary tumour virus long terminal redundancy. J. Virol. 37, 226-238.

Gottlieb, M.S., Schroff, R., Schanler, H.M., Weisman, J.D., Fan P.T., Wolf R.A., Saxon, A. (1981). Pneumocytis carinii pneumonia and mucosal candidiasis in previously healthy homosexual men : Evidence of a new acquired cellular immuno-deficiency. N. Engl. J. Med. 305, 1426-1431.

Hahn, B.H., Shaw, G.M., Arya, S.U., Popovic, M., Gallo, R.C. and Wong-Stall, F. (1984). Molecular cloning and characterization of the HTLV-III virus associated with AIDS. Nature 312, 166-169.

Harris, J.D., Scott, J.V., Taylor, B., Brahic, M., Stowring, L., Ventura, P., Haase, A.T. and Peluso, R. (1981). Visna virus DNA : discovery of a novel gapped structure. Virology 113, 573-583.

Kiyokawa, T., Yoshikura, H., Hattori, S., Secki, M. and Yoshida, M. (1984). Envelope proteins of human T-cell leukemia virus : expression in Escherischia coli and its application to studies of env gene functions. Proc. Natl. Acad. Sci. USA 81, 6202-6206.

Klatzmann, D., Barré-Sinoussi, F., Nugeyre, M.T., Dauguet, C., Vilmer, E., Griscelli, C., Brun-Vezinet, F., Rouzioux, C., Gluckman, J.C., Chermann, J.C. and Montagnier, L. (1984). Selective tropism of lymphadenopathy associated virus (LAV) for helper-inducer T-lymphocytes. Sciences 225, 59-63.

Kozak, M. (1984). Compilation and analysis of sequences upstream from the transcriptional start site in eucaryotic mRNAs. Nucl. Acids Res. 12, 857-872.

Levy, J.A., Hoffman, A.D., Kramer, S.M., Lanois, J.A., Shimabukuro, J.M. and Oskiro, L.S. (1984). Isolation of lymphocytopathic retroviruses from San Francisco patients with AIDS. Science 225,840-842.

Masur, H., Michelis, M.A., Greene, J.B., Onovato, I., Van de Stowe, R.A., Holzman, R.S., Wormser, G., Brettman, L., Lange, M., Murray, H.W., Cunningham-Rundles, S. (1981). An outbreak of community-acquired pneumocystis carinii pneumonia : Initial manifestation of cellular immune dysfunction. N. Engl. J. Med. 305, 1431-1438.

Misra, T.K., Grandgenett, D.P. and Parsons, J.T. (1982). Avian retrovirus pp32 DNA-binding protein. I. Recognition to specific sequences on retrovirus DNA terminal repeats. J. Virol. 44,330-343.

Montagnier, L., et al., (1984). A new human T-lymphotropic retrovirus : characterization and possible role in lymphadenopathy and acquired immune deficiency syndromes. In human T-cell leukemia/lymphoma viruses. R.C. Gallo, M. Essex and L. Gross, eds. (Cold Spring Laboratory, New-York), pp 363-370.

Oroszlan, S., Copeland, T.D., Kalyanaraman, V.S., Sarngadharan, M.G., Schultz, A.M. and Gallo, R.C. (1984). Chemical analysis of human T-cell leukemia virus structural proteins. In HTLVS (R.C. Gallo, M.E. Essex and L. Gross, eds) Cold Spring Laboratory, New-York, pp 101-110.

Piot, P., Quinn, T.C., Taelmann, H., Feinsod, F.M. et al., (1984). Acquired immunodeficiency syndrome in a heterosexual population in Zaire. Lancet II,65-69.

Popovic, M., Sarngadharan, M.G., Read, E. and Gallo, R.C. (1984). Detection, isolation, and continuous production of cytopathic retroviruses (HTLV-III) from patients with AIDS and pre-AIDS. Science 224,497-500.

Querat, G., Barban, N., Sauze, N., Filippi, P., Vigne, R., Russo, P. and Vitu, C. (1984). Highly lytic and persistent lentiviruses naturally present in sheep with progressive pneumonia are genetically distinct. J. Virol. 52, 672-679.

Raba, M., Limburg, K., Burghagen, M., Katze, J.R., Simsek. M., Heckman, J.E., Rajbhandary, U.L., and Gross, H.J. (1979). Nucleotide sequence fo three isoaccepting lysine tRNAs from rabbit liver and SV40-transformed mouse fibroblasts. Eur. J. Biochem. 97,305-318.

Rice, N.R., Stephens, R.M., Couez, D., Deschamps, J., Kettmann, R., Burny, A., and Gilden, R.V. (1984). The nucleotide sequence of the env gene and post-env region of bovine leukemia virus. Virology 138,82-93.

Sagata, N., Yasunaga, T., Ogawa, Y., Tsuzuku-Kawamura, J. and Ikawa, Y. (1984). Bovine leukemia virus : Unique structural features of its long terminal repeats and its evolutionary relationship to human T-cell leukemia virus. Proc. Natl. Acad. Sci. USA 81, 4741-4745.

Sanger, F., Nicklen, S. and Coulsen, A.R. (1977). DNA sequencing with chain terminating inhibitors. Proc. Natl. Acad. Sci. USA 74, 5463-5467.

Schwartz, D.E., Tizard, R. and Gilbert, W. (1983). Nucleotide sequence of Rous sarcoma virus. Cell 32,853-869.

Schüpbach, J., Popovic, M., Gilden, R.V., Gonda, M.A., Sarngadharan, M.G. and Gallo, R.C. (1984). Serological analysis of a subgroup of human T-lymphotropic retroviruses (HTLV-III) associated with AIDS. Science 224, 503-505.

Seiki, M., Hattori, S., Hirayama, Y. and Yoshida, M. (1983). Human adult T-cell leukemia virus : complete nucleotide sequence of the provirus genome integrated in leukemia cell DNA. Proc. Natl. Acad. Sci. USA 80, 3618-3622.

Shaw, G.M., Hahn, B.H., Arya, S.K., Groopman, J.E., Gallo, R.C. and Wong-Staal, F. (1984). Molecular characterization of human T-cell leukemia (lymphotropic) virus type III in the Acquired Immune Deficiency Syndrome. Science 226, 1165-1171.

Shimotohno, k., and Temin, H.M. (1982). Spontaneous variation and synthesis in the U3 region of the long terminal repeat of avion retroviruses. J. Virol. 41,1636171.

Shimotohno, K., Golde, D.M., Miwa, M., Sugimura, T. and Chen, I.S.Y. (1984). Nucleotide sequence analysis fo the long terminal repeat of human T-cell leukemia virus type II. Proc. Natl. Acad. Sci. USA 81, 1079-1083.

Shinnick, T.M., Lerner, R.A. and Sutcliffe, J.G. (1981). Nucleotide sequence of Moloney murine leukemia viruse. Nature 293, 543-548.

Srinivasan, A., Reddy, E.P., Dunn, C.Y. and Aaronson, S.A. (1984). Molecular dissection of transcriptional control elements with the long terminal repeat of retrovirus. Science 223, 286-289.

Staden, R. (1982). Automation of the computer handling of gel reading data produced by the shotgun method of DNA sequencing. Nucl. Acids. Res. 10, 4731-4751.

Temin. H. (1981). Structure, variation and synthesis of retrovirus long terminal repeat. Cell 27, 1-3.

Weinberg, R.A. (1982). Fewer and fewer oncogenes. Cell 30, 3-9.

## Claims

1. A purified product having the following characteristics:
a) it contains the polypeptidic backbone of a LAV retrovirus envelope glycoprotein substantially free of non viral constituents, particularly cellular constituents, wherein said glycoprotein has a molecular weight of the order of 110,000 daltons as measurable by its migration distance in a migration system in comparison with the distances of migration of standard proteins having known molecular weights in the same migration system, said glycoprotein providing upon treatment with endoglycosidases and subsequent removal of the sugar residues a protein having a molecular weight of about 90,000 daltons as measurable in the same migration system;
b) it possesses the capacity of being recognized by serums of human origin and containing antibodies against the LAV virus.

2. The purified product of claim 1 which has a molecular weight of about 110,000-120,000 daltons as measurable on a 12.5% polyacrylamide gel, under a voltage of 18V for 18 hours, wherein said standard proteins consist of:
- lysozyme-(¹⁴C)-methyl (MW: 14,300),
- carbon dioxide-(¹⁴C)-methyl (MW: 30,000),
- ovalbumin-(¹⁴C)-methyl (MW: 46,000),
- bovin albumin serum (¹⁴C)-methyl (MW: 69,000),
- phosphorylase b-(¹⁴C)-methyl (MW: 92,500),
- myosine-(¹⁴C)-methyl (MW: 200,000).

3. The glycoprotein of claim 2 which forms complexes with concanavaline A, said complex being dissociatable in the presence of O-methyl-α-D-mannopyranoside.

4. The glycoprotein of claim 2 or 3, which binds to lectins.

5. The purified product of claim 1, which is said viral envelope glycoprotein freed of sugar residues separable from said viral envelope glycoprotein under the action of endoglycosidases.

6. The purified product of any one of claims 1 to 5 which contains the polypeptidic backbone of the polypeptide encoded by the nucleic acid fragment extending between nucleotide numbered 5746 and nucleotide numbered 8208 of fig. 4.

7. The purified product of any one of claims 1 to 5. which contains the polypeptidic backbone of the polypeptide encoded by the nucleic acid fragment extending between nucleotide numbered 5746 and nucleotide numbered 8350 of fig. 4.

8. The purified product of any one of claims 1 to 5 which contains the polypeptidic backbone of the polypeptide encoded by the nucleic acid fragment extending between nucleotide numbered 5767 and nucleotide numbered 8208 of fig. 4.

9. The purified product of any one of claims 1 to 5 which contains the polypeptidic backbone of the polypeptide encoded by the nucleic acid fragment extending between nucleotide numbered 5767 and nucleotide numbered 8350 of fig. 4.

10. The purified product of any of claims 5 to 9 which is the expression product of a cDNA (orf-env) corresponding to the nucleic acid fragment between the nucleotides numbered 5746 and 8208 of fig.4 or between the nucleotides numbered 5746 and 8350 of fig.4 or between the nucleotides numbered 5767 and 8208 of fig.4 or between the nucleotides numbered 5767 and 8350 of fig.4 in an appropriate cell host.

11. A purified product which contains the polypeptide defined in claim 10 in a fused form with other polypeptidic fragments normally foreign to the LAV proteins and which either are immunogenically inert or do not interfere with the immunogenic properties of said polypeptide.

12. Process for obtaining the purified product of any one of claims 1 to 4, which process comprises starting from a LAV virus obtained from the supernatant of a culture of cells infected therewith and purified under conditions such that it still carries substantial proportion of envelope antigens, lysing the virus, recovering the antigens released in the supernatant and separating the purified product from other components of the lysate.

13. The process of claim 12 wherein the starting virus has been purified by centrifugation operations, which centrifugation operations comprise particularly a first centrifugation of said supernatant at an angular centrifugation velocity, particularly of 10,000 rpm, enabling the removal of non-viral constituents, more particularly of cellular constituents, then a second centrifugation at higher angular velocity, particularly at 45,000 rpm, to obtain the precipitation of said starting virus.

14. A process of claim 12 or 13 which comprises further treating the purified product obtained with an endoglycosidase and recovering the purified product freed of glycosidation residues.

15. The process of any of claims 12 to 14 in which the separation of the purified product is by contacting the expression products of said cell with antibodies specifically recognizing a glycoprotein or protein according to any one of claims 1 to 10.

16. A method for the diagnostic of antibodies to the LAV virus in a biological fluid, particularly a human serum, which comprises contacting said biological fluid with the product of any of claims 1 to 11 under conditions suitable for enabling a complex between said antibodies and said product to be formed and detecting said complex as indicative of the presence of said antibodies in said biological fluid.

17. A kit for in vitro detection of antibodies against the LAV virus, which kit comprises the purified product of any of claims 1 to 11, suitable buffers and anti-human immunoglobulins, which anti-human immunoglobulins are labelled either by an immunofluorescent molecule or by an enzyme and, in the last instance, a substrate hydrolysable by the enzyme for providing a signal, particularly modified absorption of a radiation, at least in a determined wavelength.

18. Antibodies specifically directed against the purified product of any of claims 1 to 11 and which specifically recognizes said purifed product or smaller peptides or fragments thereof and containing one of its antigenic sites or epitopes.

19. The antibodies of claim 18 which consist of monoclonal antibodies.

20. A cDNA derived from the LAV genome which extends between nucleotide numbered 5746 and nucleotide numbered 8208 of fig. 4.

21. A cDNA derived from the LAV genome which extends between nucleotide numbered 5746 and nucleotide numbered 8350 of fig. 4.

22. A cDNA derived from the LAV genome which extends between nucleotide numbered 5767 and nucleotide numbered 8208 of fig. 4.

23. A cDNA derived from the LAV genome which extends between nucleotide numbered 5767 and nucleotide numbered 8350 of fig. 4.

24. A recombinant vector which contains the cDNA of claim 21 and which is adapted to transform coresponding microorganisms or cells, particularly procaryotic cells such as E. coli, or eukaryotic cells, particularly yeast or higher eucaryotic cells, e.g. mammalian cells, and to permit the amplification of said cDNA therein.

25. A cell culture transformed by the vector of claim 24.

## Patentansprüche

1. Gereinigtes Produkt mit den folgenden Merkmalen:
a) es enthält das polypeptidische Gerüst eines LAV-Retrovirus-Hüllglykoproteins, das im wesentlichen von nicht-viralen Bestandteilen, insbesondere zellulären Bestandteilen frei ist, wobei dieses Glykoprotein ein Molekulargewicht in der Größenordnung von 110 000 Daltons aufweist, meßbar durch dessen Wanderungsentfernung in einem Wanderungssystem im Vergleich mit den Wanderungsentfernungen von Standardproteinen mit bekannten Molekulargewichten im gleichen Wanderungs-System, wobei das Glykoprotein bei der Behandlung mit Endoglykosidasen und nachfolgender Entfernung der Zuckerreste ein Protein ergibt, das ein Molekulargewicht von etwa 90 000 Dalton, meßbar im gleichen Wanderungssystem, besitzt;
b) es besitzt die Fähigkeit, durch Seren menschlichen Ursprungs erkannt zu werden und Antikörper gegen das LAV-Virus zu enthalten.

2. Gereinigtes Produkt nach Anspruch 1, mit einem Molekulargewicht von etwa 110 000 bis 120 000 Dalton, meßbar auf einem 12,5%-igen Polyacrylamidgel in 18 Stunden bei einer Spannung von 18 V, wobei die Standardproteine bestehen aus:
- Lysozym-(¹⁴C)-methyl (MG: 14 300),
- Kohlendioxid-(¹⁴C)-methyl (MG: 30 000),
- Ovalbumin-(¹⁴C)-methyl (MG: 46 000),
- Rinderalbuminserum (¹⁴C)-methyl (MG: 69 000),
- Phosphorylase b-(¹⁴C)-methyl (MG: 92 500),
- Myosin-(¹⁴C)-methyl (MG: 200 000).

3. Glykoprotein nach Anspruch 2, das Komplexe mit Concanavalin A bildet, wobei dieser Komplex in Gegenwart von O-Methyl-α-D-mannopyranosid dissoziierbar ist.

4. Glykoprotein nach Anspruch 2 oder 3, das an Lectine bindet.

5. Gereinigtes Produkt von Anspruch 1, welches das virale Hüllglykoprotein ist, das von Zuckerresten befreit ist, die von diesem viralen Hüllglykoprotein unter der Wirkung von Endoglykosidasen abtrennbar sind.

6. Gereinigtes Produkt nach einem der Ansprüche 1 bis 5, das das polypeptidische Gerüst des Polypeptids enthält, das durch das Nucleinsäurefragment codiert wird, welches sich zwischen dem Nucleotid Nr. 5746 und dem Nucleotid Nr. 8208 von Fig. 4 befindet.

7. Gereinigtes Produkt nach einem der Ansprüche 1 bis 5, das das polypeptidische Gerüst des Polypeptids enthält, das durch das Nucleinsäurefragment codiert wird, welches sich zwischen dem Nucleotid der Nr. 5746 und dem Nucleotid der Nr. 8350 von Fig. 4 befindet.

8. Gereinigtes Produkt nach einem der Ansprüche 1 bis 5, das das polypeptidische Gerüst des Polypeptids enthält, das durch das Nucleinsäurefragment codiert wird, welches sich zwischen dem Nucleotid der Nr. 5767 und dem Nucleotid der Nr. 8208 von Fig. 4 befindet.

9. Gereinigtes Produkt nach einem der Ansprüche 1 bis 5, das das polypeptidische Gerüst des Polypeptids enthält, das durch das Nucleinsäurefragment codiert wird, welches sich zwischen dem Nucleotid der Nr. 5767 und dem Nucleotid der Nr. 8350 von Fig. 4 befindet.

10. Gereinigtes Produkt nach einem der Ansprüche 5 bis 9, das das Expressionprodukt einer cDNA (orf-env), die dem Nucleinsäurefragment zwischen den Nucleotiden der Nr. 5746 und 8208 von Fig. 4 oder zwischen den Nucleotiden der Nr. 5746 und 8350 von Fig. 4 oder zwischen den Nucleotiden der Nr. 5767 und 8208 von Fig. 4 oder zwischen den Nucleotiden der Nr. 5767 und 8350 von Fig. 4 entspricht, in einer geeigneten Wirtszelle darstellt.

11. Gereinigtes Produkt, das das in Anspruch 10 definierte Polypeptid in einer mit anderen polypeptidischen Fragmenten fusionierten Form enthält, welche normalerweise in den LAV-Proteinen nicht vorkommen und welche entweder nicht immunogen aktiv sind oder die immmogenen Eigenschaften des genannten Polypeptids nicht beeinträchtigen.

12. Verfahren zur Herstellung des gereinigten Produkts nach einem der Ansprüche 1 bis 4, das das Ausgehen von einem LAV-Virus, das aus dem Überstand einer Zellkultur erhalten wird, die damit infiziert ist, und das unter solchen Bedingungen gereinigt ist, daß es noch einen wesentlichen Teil der Hüllantigene trägt, das Lysieren des Virus, die Gewinnung des Antigens, welches in den Überstand freigesetzt wird, und das Abtrennen des gereinigten Produkts von anderen Bestandteilen des Lysats umfaßt.

13. Verfahren nach Anspruch 12, bei dem das Ausgangsvirus durch Zentrifugiervorgänge gereinigt worden ist, wobei die Zentrifugiervorgänge insbesondere eine erste Zentrifugierung des Überstands bei einer Zentrifugier-Winkelgeschwindigkeit, insbesondere von 10 000 U/min, welche die Entfernung nicht-viraler Bestandteile, insbesondere zellulärer Bestandteile ermöglicht, und anschließend eine zweite Zentrifugierung bei höherer Winkelgeschwindigkeit, insbesondere von 45 000 U/min, zur Ausfällung des Ausgangsvirus umfassen.

14. Verfahren nach Anspruch 12 oder 13, umfassend weiteres Behandeln des erhaltenen gereinigten Produkts mit einer Endoglykosidase und Gewinnung des gereinigten Produkts, das von Glykosidierungsresten befreit ist.

15. Verfahren nach einem der Ansprüche 12 bis 14, bei dem die Abtrennung des gereinigten Produkts durch Inkontaktbringen der Expressionsprodukte dieser Zelle mit Antikörpern geschieht, die ein Glykoprotein oder Protein nach einem der Ansprüche 1 bis 10 spezifisch erkennen.

16. Verfahren zur Diagnose von Antikörpern gegen das LAV-Virus in einer biologischen Flüssigkeit, insbesondere einem menschlichen Serum, das das Inkontaktbringen der biologischen Flüssigkeit mit dem Produkt nach einem der Ansprüche 1 bis 11 unter Bedingungan, die geeignet sind, daß sich ein Komplex zwischen den Antikörpern und dem Produkt bilden kann, und den Nachweis des Komplexes als Indikator für die Anwesenheit der Antikörper in der biologischen Flüssigkeit umfaßt.

17. Besteck (Kit) für den in vitro-Nachweis von Antikörpern gegen den LAV-Virus, umfassend das gereinigte Produkt nach einem der Ansprüche 1 bis 11, geeignete Puffer und Antihuman-Immunoglobuline, die entweder durch ein immunofluoreszierendes Molekül oder durch ein Enzym markiert sind, und im letzten Fall ein durch das Enzym hydrolysierbares Substrat, wodurch ein Signal erhalten wird, insbesondere eine modifizierte Absorption einer Strahlung, mindestens bei einer bestimmten Wellenlänge.

18. Antikörper, die spezifisch gegen das gereinigte Produkt nach einem der Ansprüche 1 bis 11 gerichtet sind und spezifisch dieses gereinigte Produkt erkennen oder kleinere Peptide oder Fragmente davon, die eine ihrer antigenen Stellen oder Epitope enthalten.

19. Antikörper nach Anspruch 18, die aus monoclonalen Antikörpern bestehen.

20. cDNA, die vom LAV-Genom abgeleitet ist und sich vom Nucleotid der Nr. 5746 bis zum Nucleotid der Nr. 8208 von Fig. 4 erstreckt.

21. cDNA, die vom LAV-Genom abgeleitet ist und sich vom Nucleotid der Nr. 5746 bis zum Nucleotid der Nr. 8350 von Fig. 4 erstreckt.

22. cDNA, die vom LAV-Genom abgeleitet ist und sich vom Nucleotid der Nr. 5767 bis zum Nucleotid der Nr. 8208 von Fig. 4 erstreckt.

23. cDNA, die vom LAV-Genom abgeleitet ist und sich vom Nucleotid der Nr. 5767 bis zum Nucleotid der Nr. 8350 von Fig. 4 erstreckt.

24. Rekombinanter Vektor, der die cDNA von Anspruch 21 enthält und der zur Transformation entsprechender Mikroorganismen oder Zellen, insbesondere prokaryotischer Zellen, wie E. coli oder eukaryotischer Zellen, insbesondere Hefe oder höhere eukaryotische Zellen, wie Säugerzellen, und zur Ermöglichung einer Amplifikation der cDNA darin angepaßt ist.

25. Zellkultur, die durch den Vektor von Anspruch 24 transformiert ist.

## Revendications

1. Un produit ayant les caractéristiques suivantes :
a) il contient l'ossature polypeptidique d'une glycoprotéine d'enveloppe de rétrovirus LAV sensiblement exempte de constituants non viraux, particulièrement de constituants cellulaires, dans lequel ladite glycoprotéine a un poids moléculaire de l'ordre de 110.000 daltons tel qu'il est mesurable par sa distance de migration dans un système de migration par comparaison avec les distances de migration dans le même système de migration de protéines-étalons ayant des poids moléculaires connus, ladite glycoprotéine fournissant, après traitement avec des endoglycosidases et séparation subséquente des groupes saccharidiques, une protéine ayant un poids moléculaire de l'ordre de 90.000 daltons, tel qu'il est mesurable dans le même système de migration ;
b) il possède la capacité d'être reconnu par des sérums d'origine humaine et contenant des anticorps contre le virus LAV.

2. Le produit purifié de la revendication 1 qui a un poids moléculaire de l'ordre de 110.000-120.000 daltons, comme il peut être mesuré dans un gel de polyacrylamide à 12,5%, sous une tension de 12.000 volts pendant 18 heures, lesdites protéines-étalons consistant en :
- lysozyme-(¹⁴C)-méthyle (PM : 14.300),
- dioxyde de carbone-(¹⁴C)-méthyle (PM : 30.000),
- ovalbumine-(¹⁴C)-méthyle (PM : 46.000),
- sérum albumine bovine-(¹⁴C)-méthyle (PM : 69.000),
- phosphorylase b-(¹⁴C)-méthyle (PM : 92.500),
- myosine-(¹⁴C)-méthyle (PM : 200.000).

3. La glycoprotéine de la revendication 2 qui forme des complexes avec la concanavaline A, ledit complexe pouvant être dissocié en présence de O-méthyl-α-D-mannopyranoside.

4. La glycoprotéine de la revendication 2 ou 3, qui se fixe sur les lectines.

5. Le produit purifié de la revendication 1, qui est ladite glycoprotéine d'enveloppe virale débarrassée des résidus saccharidiques qui peuvent être séparés de ladite glycoprotéine d'enveloppe virale sous l'action des endoglycosidases.

6. Le produit purifié de l'une quelconque des revendications 1 à 5, qui contient l'ossature polypeptidique du polypeptide codé par le fragment d'acide nucléique qui s'étend entre le nucléotide numéroté 5746 et le nucléotide numéroté 8208 de la figure 4.

7. Le produit purifié de l'une quelconque des revendications 1 à 5, qui contient l'ossature polypeptidique du polypeptide codé par le fragment d'acide nucléique qui s'étend entre le nucléotide numéroté 5746 et le nucléotide numéroté 8350 de la figure 4.

8. Le produit purifié de l'une quelconque des revendications 1 à 5, qui contient l'ossature polypeptidique du polypeptide codé par le fragment d'acide nucléique qui s'étend entre le nucléotide numéroté 5767 et le nucléotide numéroté 8208 de la figure 4.

9. Le produit purifié de l'une quelconque des revendications 1 à 5, qui contient l'ossature polypeptidique du polypeptide codé par le fragment d'acide nucléique qui s'étend entre le nucléotide numéroté 5767 et le nucléotide numéroté 8350 de la figure 4.

10. Le produit purifié de l'une quelconque des revendications 5 à 9 qui est le produit d'expression d'un ADNc (orf-env) correspondant au fragment d'acide nucléique entre les nucléotides numérotés 5746 et 8208 de la figure 4 ou entre le nucléotide numéroté 5746 et 8350 de la figure 4 ou entre les nucléotides numérotés 5767 et 8208 ou entre les nucléotides numérotés 5767 et 8350 de la figure 4, dans un hôte cellulaire appriorié.

11. Un produit purifié qui contient le polypeptide défini dans la revendication 10 à l'état recombiné avec d'autres fragments polypeptidiques normalement étrangers au protéines de LAV et qui, soit sont immunogéniquement inertes, soit n'interfèrent pas avec les propriétés immunogéniques dudit polypeptide.

12. Procédé pour obtenir le produit purifié de l'une quelconque des revendications 1 à 4, ce procédé comprenant, partant d'un virus LAV obtenu à partir d'un surnageant de culture de cellules infectées avec ce virus et purifié dans des conditions telles qu'il comporte encore des proportions substantielles d'antigènes d'enveloppe, la lyse du virus, la récupération des antigènes libérés dans le surnageant et la séparation du produit purifié à partir des autres constituants du lysat.

13. Le procédé de la revendication 12, dans lequel le virus initial a été purifié par des opérations de centrifugation, lesdites opérations de centrifugation comprenant notamment une première centrifugation dudit surnageant à une vitesse angulaire de centrivugation, notamment de 10.000 rpm, permettant la séparation des constituants non viraux, particulièrement des constituants cellulaires, puis une seconde centrifugation à une vitesse angulaire plus élevée, notamment de 45,000 rpm, pour obtenir la précipitation dudit virus initial.

14. Procédé selon la revendication 12 ou 13 qui comprend le traitement supplémentaire du produit purifié obtenu avec une endoglycosidase et la récupération du produit purifié rendu exempt de groupe de glucosydation.

15. Le procédé selon l'une quelconque des revendications 12 à 14 dans lequel la séparation du produit purifié est effectuée par contact des produits d'expression de ladite cellule avec des anticorps reconnaissant spécifiquement une glycoprotéine ou une protéine selon l'une quelconque des revendications 1 à 10.

16. Une méthode pour le diagnostic d'anticorps contre le virus LAV dans un fluide biologique, particulièrement un sérum humain, qui comprend la mise en contact dudit fluide biologique avec le produit de l'une quelconque des revendications 1 à 11, dans des conditions appropriées pour permettre la formation d'un complexe contre lesdits anticorps et ledit produit, et la détection dudit complexe qui est indicatif de la présence desdits anticorps dans ledit fluide biologique.

17. Kit pour la détection in vitro d'anticorps contre le virus LAV, ledit kit comportant le produit purifié de l'une quelconque des revendications 1 à 11, des tampons appropriés et des immunoglobulines anti-humaines, lesdites immunoglobulines anti-humaines étant marquées, soit par une molécule immunofluorescente, soit par une enzyme et, dans le dernier cas, un substrat hyudrolysable par l'enzyme pour fournir un signal, particulièrement une absorption modifiée d'une radiation, au moins dans une longueur d'onde déterminée.

18. Anticorps dirigés spécifiquement contre le produit purifié de l'une quelconque des revendications 1 à 11 et qui reconnaissent spécifiquement ledit produit purifié ou des peptides plus petits ou fragments de celui-ci et qui contiennent l'un de leurs sites antigéniques ou épitopes.

19. Les anticorps de la revendication 18 qui consistent en des anticorps monoclonaux.

20. Un ADNc dérivé du génome de LAV qui s'étend entre le nucléotide numéroté 5746 et le nucléotide numéroté 8208 de la figure 4.

21. Un ADNc dérivé du génome de LAV qui s'étend entre le nucléotide numéroté 5746 et le nucléotide numéroté 8350 de la figure 4.

22. Un ADNc dérivé du génome de LAV qui s'étend entre le nucléotide numéroté 5767 et le nucléotide numéroté 8208 de la figure 4.

23. Un ADNc dérivé du génome de LAV qui s'étend entre le nucléotide numéroté 5767 et le nucléotide numéroté 8350 de la figure 4.

24. Un vecteur recombinant qui contient l'ADNc de la revendication 21 et qui est adapté à la transformation des microorganismes ou cellules correspondants, particulièrement des cellules procaryotes telles que E.coli, ou des cellules eucaryotes, particulièrement des cellules de levures d'eucaryotes supérieurs, par exemple des dellules de mammifères, et à permettre l'amplification dudit ADNc dans lesdits microorganismes ou cellules.

25. Une culture cellulaire transformée par le vecteur de la revendication 24.
